# EUROPEAN PATENT SPECIFICATION

(11) **EP 2 181 673 B1**
(45) Date of publication and mention of the grant of the patent: **07.12.2011**
(21) Application number: 09174095.1
(22) Date of filing: 26.10.2009
(51) Int. Cl.: A61F 2/30, A61B 17/16, A61B 17/17

(54) **Knee prosthesis kit with a winged metaphyseal sleeve**
Knieprothesesatz mit einer Metaphysenhülse mit Flügeln
Kit de prothèse du genou pour un manchon métaphysaire ailé

(30) Priority: 31.10.2008 US 110174 P
(43) Date of publication of application: 05.05.2010
(73) Proprietor: DePuy Products, Inc., Warsaw, IN 46581 (US)
(72) Inventor: Deruntz, Stephanie, Pierceton, IN 46562 (US); Haas, Brian D., Englewood, CO 80110 (US); Haidukewych, George J., Orlando, FL 32819 (US); Koenemann, Jeffery L., Plymouth, IN 46563 (US); Vendrely, Timothy G., Fort Wayne, IN 46805 (US)
(74) Representative: Belcher, Simon James

(56) References cited:
- EP-A2- 0 980 679
- US-A- 4 790 852
- US-A1- 2003 171 756
- US-A1- 2004 153 081

## Description

The present invention relates generally to prosthetic joints, and more particularly to a modular prosthetic knee joint system that includes a metaphyseal component and an instrument preparing the bone to receive the metaphyseal component.

The knee joint basically consists of the bone interface of the distal end of the femur and the proximal end of the tibia. Appearing to cover or at least partially protect this interface is the patella, which is a sesamoid bone within the tendon of the long muscle (quadriceps) on the front of the thigh. This tendon inserts into the tibial tuberosity and the posterior surface of the patella is smooth and glides over the femur.

The femur is configured with two knob like processes (the medial condyle and the lateral condyle) which are substantially smooth and which articulate with the medial plateau and the lateral plateau of the tibia, respectively. The plateaus of the tibia are substantially smooth and slightly cupped thereby providing a slight receptacle for receipt of the femoral condyles.

When the knee joint is damaged whether as a result of an accident or illness, a prosthetic replacement of the damaged joint may be necessary to relieve pain and to restore normal use to the joint. Typically the entire knee joint is replaced by means of a surgical procedure that involves removal of the surfaces of the corresponding damaged bones and replacement of these surfaces with prosthetic implants. This replacement of a native joint with a prosthetic joint is referred to as a primary total-knee arthroplasty.

On occasion, the primary knee prostheses fails. Failure can result from many causes, including wear, aseptic loosening, osteolysis, ligamentous instability, arthrofibrosis and patellofemoral complications. When the failure is debilitating, revision knee surgery may be necessary. In a revision, the primary knee prosthesis is removed and replaced with components of a revision prosthetic knee system.

Knee implant systems for both primary and revision applications are available from a variety of manufacturers. Examples of commercially available knee implant systems include those sold by DePuy Orthopaedics Inc under the trade marks PFC SIGMA, LCS and S-ROM. Each of these orthopaedic knee systems includes several components, some appropriate for use in primary knee arthroplasty and some appropriate for use in revision surgery.

The system sold by DePuy Orthopaedics Inc under the trade mark LPS is for use in cases of severe trauma and disease. In such cases, the trauma or disease can lead to significant amounts of bone loss. The LPS system provides components that can replace all or significant portions of a particular bone, such as the femur. Details of the system are disclosed in US-A-2003/0204267.

In some patients, the metaphysis of the bone near the joint presents cavitary defects that are not completely filled by standard knee implants. The presence of such metaphyseal defects can result in loosening of the prosthetic implant over time, compromising the stability of the prosthetic implant and frequently requiring revision of the prosthetic implant.

To fill metaphyseal cavitary defects, knee systems with modular metaphyseal sleeves have been provided. Such sleeves are disclosed in US-A-2006/0030945, US-A-2005/0107883, US-7291174, US-6171342, US-5824097, US-5782921 and US-4634444. Such sleeves have been used in commercially available prosthetic knee implant systems, such as those referred to above which are sold under the trade marks PFC SIGMA, LCS and S-ROM.

Examples of modular sleeves which have been used in hip implant systems are disclosed in US-6264699 and US-4790852. Such hip sleeves have been used in commercially available prosthetic hip implant systems, such as that the system referred to above which is sold under the trade mark S-ROM.

In knee systems with modular metaphyseal sleeves, the conventional shape of many of the sleeves is generally an elliptical cone with a large ellipse profile close to the joint line tapering down to a smaller elliptical or circular profile at the termination of the component distal to the joint line. Generally, the sleeves have a terraced or stepped outer surface and an inner channel for frictional fixation to another component. This geometry fills cavitary defects in the metaphysis, allows for a wider surface area for load transfer through the joint and provides rotational stability for the articulating components of the prosthesis. The current sleeve geometry is conducive to preparation of the bone through broaching.

Generally, broaches are tapered tools used to shape or enlarge a cavity in the bone by impacting the broach into the metaphysis of the bone. Typical broaches in knee implant instrument sets have an outer shape generally conforming to the shape of the metaphyseal sleeve component. Generally, the cavity prepared by the broach is preferably aligned with the intramedullary canal of the bone so that the implant that is placed in the cavity is properly positioned.

In some patients, the bone presents regions of hard sclerotic bone adjacent to the softer bone of the metaphysis. In such patients, the hard sclerotic bone may force the broaches to remove more bone in softer areas and less bone in harder areas, resulting in the implants being positioned in an undesirable location. This situation is more common at the distal end of the femur where posterior bone is typically harder than the anterior bone. Conventional broaching may tend to cause the prepared cavity, and hence the implant, to be positioned too anterior, creating a flexion gap and making balancing of the joint more difficult. In addition, improper implant placement can adversely affect the long-term performance and survivorship of the prosthesis.

Moreover, in some patients, metaphyseal defects may be single sided: there may be a medial or lateral defect that requires use of metaphyseal sleeve but the opposite side may have healthier bone. To use a conventional symmetric sleeve in such cases would require the sacrifice of some healthy bone that could have been left in place to provide support for the prosthesis. Some have attempted to address single-side defects through use of augments; however, such systems address only peripheral defects and do not account for situations where the peripheral bone is intact but there is a central void

EP-A-980679 discloses a femoral component of a knee joint prosthesis which includes a post and a sleeve which fits over the post. The outer surface of the sleeve is stepped.

Accordingly, there is a need for a knee implant system that provides a more predictable and reliable means of metaphyseal bone preparation so that the implant is properly positioned, regardless of hard sclerotic bone interference. There is also a need for a knee implant system that addresses single-side defects of the metaphysis.

This invention provides a modular knee prosthesis kit as defined in claim 1.

A femoral adapter can be mounted on the femoral component.

Preferably, the kit includes two sleeves, one of the sleeves being asymmetric and the other sleeve being symmetric. The asymmetric sleeve has a single side wing to accommodate patients with non-centralized metaphyseal defects. The symmetric sleeve has two side wings to accommodate patients with centralized metaphyseal defects.

Accordingly, the kit can include a second sleeve which comprises a body having a central longitudinal axis which defines first and second ends, a central portion, a first side wing portion, a second side wing portion and an inner wall defining a channel along the central longitudinal axis. The channel tapers between the first and second ends. The body has an outer surface that includes a plurality of adjacent terraces. Each terrace has an outer edge around its perimeter. The outer edge of each terrace lies in a plane substantially perpendicular to the central longitudinal axis of the body. The outer edge of each terrace at the central portion of the body comprises a pair of diametrically-opposed curved portions having the same radius of curvature. The centre of curvature of each of the diametrically-opposed curved portions is along the central longitudinal axis of the body. The diametrically-opposed curved portions define vertices of the outer edge at the central portion of the body. The outer edge of each terrace at the first side wing portion of the body comprises a pair of spaced opposed straight lines connected to the diametrically-opposed curved portions and connected by a curved end segment spaced from the diametrically-opposed curved portions of the central portion. The distance between the spaced opposed lines of the outer edge at the first portion is less than the distance between the diametrically-opposed curved portions of the outer edge at the central portion of the body. The curved end segment defines a vertex of the outer edge at the first side wing portion of the body. The outer edge of each terrace at the second side wing portion of the body comprises a pair of spaced opposed straight lines connected to the diametrically-opposed curved portions of the central portion of the body and by a curved end segment spaced from the diametrically-opposed curved portions. The distance between the opposed lines being of the outer edge at the second side wing portion is less than the distance between the diametrically-opposed curved portions of the outer edge at the central portion of the body. The curved end segment defining a vertex of the outer edge at the second side wing portion of the body. Each terrace has a first transverse dimension between the vertices of the outer edge at the first portion and second portion and a second transverse dimension between the vertices of the diametrically opposed curved portions of the outer edge at the central portion. The first transverse dimension of the terrace nearest the first end is greater than the first transverse dimension of the adjacent terrace and the second transverse dimension of the terrace nearest the first end is greater than the second transverse dimension of the adjacent terrace.

Preferably, the sleeve is symmetric and the outer edge of each terrace is indented at the junctions of the diametrically-opposed curved portions of the outer edge and the spaced opposed straight lines of the outer edge.

The kit can include a milling guide and a reamer. The milling guide is used in forming a cavity to receive the sleeve, and comprises a template member, a base member and a side wall connecting the template member and the base member. The template member has an inner surface defining an elongate milling guide opening. The elongate milling guide opening has a first transverse dimension and a second transverse dimension. The first transverse dimension of the elongate milling guide opening is at least as great as the first transverse dimension of the terrace of the sleeve nearest the first end of the sleeve. The second transverse dimension is less than the first transverse dimension. The base is spaced from the template member, and includes a spherical depression aligned with the milling guide opening. The reamer has a first end, a second end and a central longitudinal axis. The first end of the reamer comprises a convex spherical surface sized and shaped to be received in the spherical depression of the base of the milling guide. The reamer is sized and shaped to extend from the base of the milling guide and through the elongate milling guide opening.

Preferably, the kit also includes a stem trial. The stem trial and the milling guide have complementary features so that the stem trial can be connected to the base member of the milling guide. Thus, the milling guide can be positioned and stabilized so that the base is aligned with the intramedullary canal of the bone.

Preferably, the kit includes a second reamer with a greater diameter than the first reamer. The second reamer is used to prepare the portion of the cavity that receives the centre portion of the sleeve. The second reamer may be connected to a stem extension to be received in the intramedullary canal so that the second reamer is aligned with the intramedullary canal in use.

Preferably, the knee prosthesis kit includes a stem extension, the second reamer and the stem extension having complementary mounting members for mounting the stem extension to the second reamer. Preferably, the central portion of the sleeve has a maximum diameter and wherein the maximum diameter of the second reamer is the same as the maximum diameter of the central portion of the sleeve.

Preferably, the spherical depression of the base is sized and shaped to limit translational movement of the convex spherical surface of the first end of the reamer to a distance less than the first transverse dimension of the elongate milling guide opening.

Preferably, the centre of the elongate milling guide opening and the centre of the spherical depression of the base lie on a central longitudinal axis. Preferably, the elongate milling guide opening has an edge including two parallel straight portions and two curved end portions, the two curved end portions connecting the two parallel straight portions, each curved end portion having a vertex, the maximum first transverse dimension of the elongate milling guide opening being at the vertices of the curved end portions, the second transverse dimension of the elongate milling guide opening being the distance between the two parallel straight portions.

Preferably, the vertices of the curved end portions of the elongate milling guide opening and the longitudinal axis of the milling guide lie in a plane, and the spherical depression of the base of the milling guide has a perimeter and opposed vertices lying in the same plane as the vertices of the curved end portions of the elongate milling guide opening and the longitudinal axis of the milling guide, and a line from each vertex of the of the curved end portions of the elongate milling guide opening to the nearest vertex of the perimeter of the spherical depression of the base defines an acute angle with the longitudinal axis of the milling guide, and the lines between the vertices define a milling envelope.

Preferably, a line tangent to the vertices of the curved end segments of the outer edge at the first side wing portion of the body defines an acute angle with the central longitudinal axis of the body, and a line tangent to the vertices of the curved end segments of the outer edge at the second side wing portion of the body defines an acute angle with the central longitudinal axis of the body, and the tangent lines define a sleeve envelope, and the milling envelope and the sleeve envelope are substantially the same.

Preferably, the outer edge of each terrace is indented at the junctions of the central portion and the first and second side wing portions.

The kit can include a milling guide for use in forming a cavity in a bone to receive a component of a knee prosthesis. The milling guide comprises a template member, a base and a side wall connecting the template member and the base member. The template member has an inner surface defining an elongate milling guide opening, the elongate milling guide opening having a first transverse dimension and a second transverse dimension. The second transverse dimension is less than the first transverse dimension. The base is spaced from the template member and includes a spherical depression aligned with the milling guide opening. The elongate milling guide opening has an edge including two parallel straight portions and two curved end portions. The two curved end portions connect the two parallel straight portions. Each curved end portion has a vertex. The maximum first transverse dimension of the elongate milling guide opening is at the vertices of the curved end portions. The second transverse dimension of the elongate milling guide opening is the distance between the two parallel straight portions. A central longitudinal axis extends from the centre of the elongate milling guide opening and through the centre of the spherical depression of the base. The vertices of the curved end portions of the elongate milling guide opening and the central longitudinal axis of the milling guide lie in a plane. The spherical depression of the base of the milling guide has a perimeter and opposed vertices lying in the same plane as the vertices of the curved end portions of the elongate milling guide opening and the longitudinal axis of the milling guide. A line from each vertex of the of the curved end portions of the elongate milling guide opening to the nearest vertex of the perimeter of the spherical depression of the base defines an acute angle with the longitudinal axis of the milling guide.

Preferably, a stem trial extends outwardly from the base along the central longitudinal axis of the milling guide.

Devices provided by the present invention can be used in a method of preparing a long bone to receive a prosthetic implant component. The method comprises preparing an elongate pilot hole aligned with and extending into the intramedullary canal of the bone. A reamer is provided, the reamer having a conical-shaped cutting portion and a shaft extending distally from the conical-shaped cutting portion. The shaft of the reamer is inserted into the pilot hole and the reamer is rotated to prepare a conical concavity aligned with the pilot hole. The reamer is then removed. A milling guide is provided. The milling guide has a template member, a base spaced from the template member, and a shaft extending distally from the base. The template member has a milling guide opening. The shaft of the milling guide is inserted into the pilot hole and the base of the milling guide is inserted into the conical concavity in the bone. A second reamer is provided. The second reamer has a cutting portion and a distal end. The second reamer is inserted through the milling guide opening and into the concavity until the distal end of the second reamer contacts the base of the milling guide. The second reamer is pivoted about the distal end on the base and the reamer is rotated to change the shape of the concavity in the bone.

Embodiments of the invention are described below by way of example with reference to the accompanying drawings, in which:
FIG. 1 is a view of the femoral components of a modular knee prosthesis kit;
FIG. 2 is a view of the tibial components of a modular knee prosthesis kit;
FIG. 3 is a side view of the single-wing metaphyseal sleeve component of the kit of FIGS. 1 and 2;
FIG. 4 is an end view of the single-wing metaphyseal sleeve component of FIG. 3;
FIG. 5 is a top plan view of the single-wing metaphyseal sleeve component of FIGS. 3 and 4;
FIG. 6 is a cross-section of the single-wing metaphyseal sleeve component of FIGS. 3 to 5, taken along line 6-6 of FIG. 3;
FIG. 7 is a cross-section of the single-wing metaphyseal sleeve component of FIGS. 3 to 5, taken along line 7-7 of FIG. 3;
FIG. 8 is a cross-section of the single-wing metaphyseal sleeve component of FIGS. 3 to 5, taken along line 8-8 of FIG. 5;
FIG. 9 is a side view of the double-wing metaphyseal sleeve component of the kit of FIGS. 1 and 2;
FIG. 10 is an end view of the double-wing metaphyseal sleeve component of FIG. 9;
FIG. 11 is a top plan view of the double-wing metaphyseal sleeve component of FIGS. 9 and 10;
FIG. 12 is a cross-section of the double-wing metaphyseal sleeve component of FIGS. 9 to 11, taken along line 12-12 of FIG. 9;
FIG. 13 is a cross-section of the double-wing metaphyseal sleeve component of FIGS. 9 to 11, taken along line 13-13 of FIG. 9;
FIG. 14 is a cross-section of the double-wing metaphyseal sleeve component of FIGS. 9 to 11, taken along line 14-14 of FIG. 11;
FIG. 15 is a front view showing an assembly of some of the components of the modular knee prosthesis kit FIGS. 1 and 2, using single-wing metaphyseal sleeves;
FIG. 16 is a side view showing an assembly of some of the components of the modular knee prosthesis kit FIGS. 1 and 2, using double-wing metaphyseal sleeves;
FIG. 17 is a front view of the assembly of FIG. 16;
FIG. 18 is a top plan view of an alternative embodiment of a double-wing metaphyseal sleeve;
FIG. 19 is a top plan view of another alternative embodiment of a double-wing metaphyseal sleeve;
FIG. 20 is a front view of a conical reamer that may be included as an instrument in the knee prosthesis kit;
FIG. 21 is a front view of another embodiment of a conical reamer that may be included as an instrument in the knee prosthesis kit;
FIG. 22 is an exploded perspective view of a milling guide, straight reamer, handle and stem trial that may be included as instruments in the knee prosthesis kit;
FIG. 23 is another exploded perspective view of the instruments of FIG. 22;
FIG. 24 is an enlarged perspective view of a portion of the milling guide of FIGS. 22 and 23;
FIG. 25 is an enlarged perspective view of a portion of the milling guide and straight reamer of FIGS. 22 and 23;
FIG. 26 is a perspective view of an assembly of the milling guide, handle and stem trial, shown with the straight reamer received within the milling guide and pivoted to one position in the milling guide opening;
FIG. 27 is another perspective view of the assembly of FIG. 26, shown with the straight reamer pivoted to another position in the milling guide opening;
FIG. 28 is another perspective view of the assembly of FIGS. 26 and 27, shown with the straight reamer pivoted to another position in the milling guide opening; and
FIG. 29 is a perspective view of an assembly of the milling guide and the handle mounted on a femoral cutting block and with the straight reamer pivoted to one position in the milling guide opening.

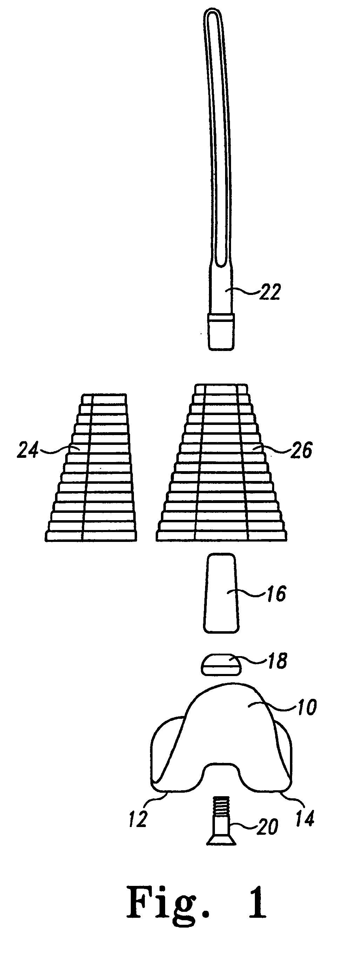

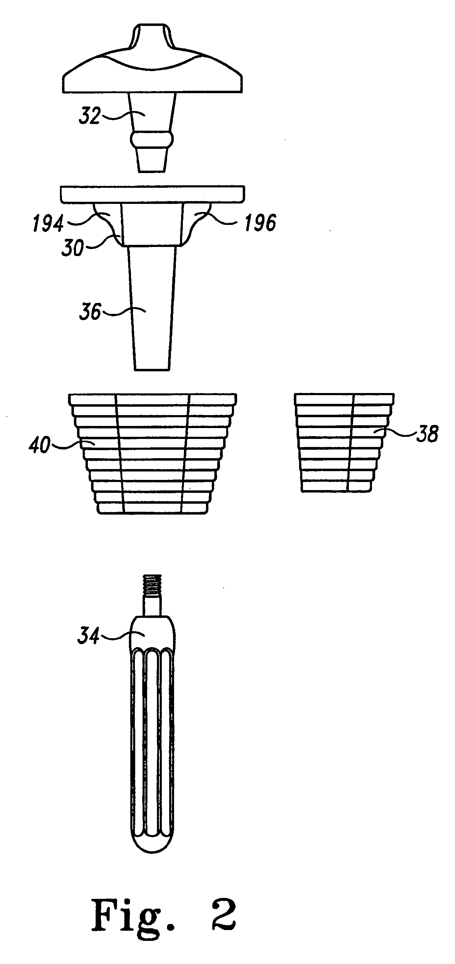

Referring to the drawings, FIGS. 1 and 2 show an example of components of a modular knee prosthesis kit. As shown in FIG. 1, on the femoral side, the kit includes a distal femoral component 10 with curved convex condylar surfaces 12, 14. The distal femoral component is a posterior stabilized component. A femoral adapter 16 is also provided, along with a collar 18 for placement between the adapter 16 and the distal femoral component 10. A bolt 20 is provided for connecting the adapter 16, collar 18 and distal femoral component 10 together. The adapter 16 has an outer surface that is smooth and tapered. A stem extension 22 is also provided. All of the above components may be standard parts of the knee system referred to above which is sold under the trade mark PFC SIGMA. The adapter may have features such as those shown in US-A-2006/0030945, or those shown in US-6171342, US-5824097, and US-5782921. The stem extension may have features other than those shown in FIG. 1, for example as disclosed in US-A-2006/ 0030945. The invention may use other types of distal femoral component or stem or other component.

As shown in FIG. 1, the femoral components of the kit also include two types of metaphyseal sleeves 24, 26. As described in more detail below, one of the metaphyseal sleeves 24 is provided for use in a single-side defect in the metaphysis of the bone and the other metaphyseal sleeve 26 is provided for use where both sides of the metaphysis are to be filled.

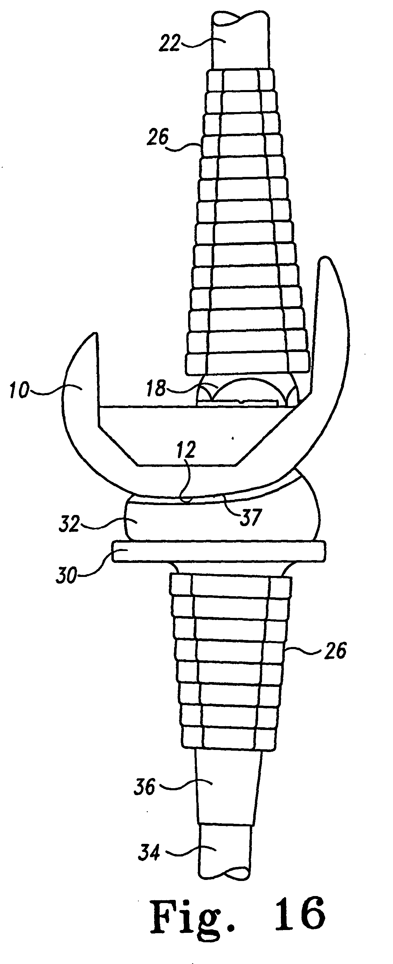

As shown in FIG. 2, on the tibial side, the kit includes a tibial tray component 30, a tibial bearing insert 32 and a stem extension 34. The tibial tray component 30 can be of the type which is sold by DePuy Orthopaedics Inc under the trade mark MBT. The tray component 30 has an integral stem portion 36 with a bore (not shown) with internal threads to which the stem extension 34 may be attached. The outer surface of the stem portion 36 has a smooth finish, and tapers away from the joint motion surface. The joint motion surface corresponds with the juncture of the curved convex condyles 12, 14 of the distal femoral component 10 and the curved concave condylar surfaces of the tibial bearing insert 32 (an example of a curved concave condylar surface 37 is shown in FIG. 16).

As shown in FIG. 2, the tibial components of the kit also include two types of metaphyseal sleeves 38, 40. As discussed below, one of the sleeves 38 is provided for use in a single-side defect in the metaphysis of the bone and the other sleeve 40 is provided for use where both sides of the metaphysis are to be filled.

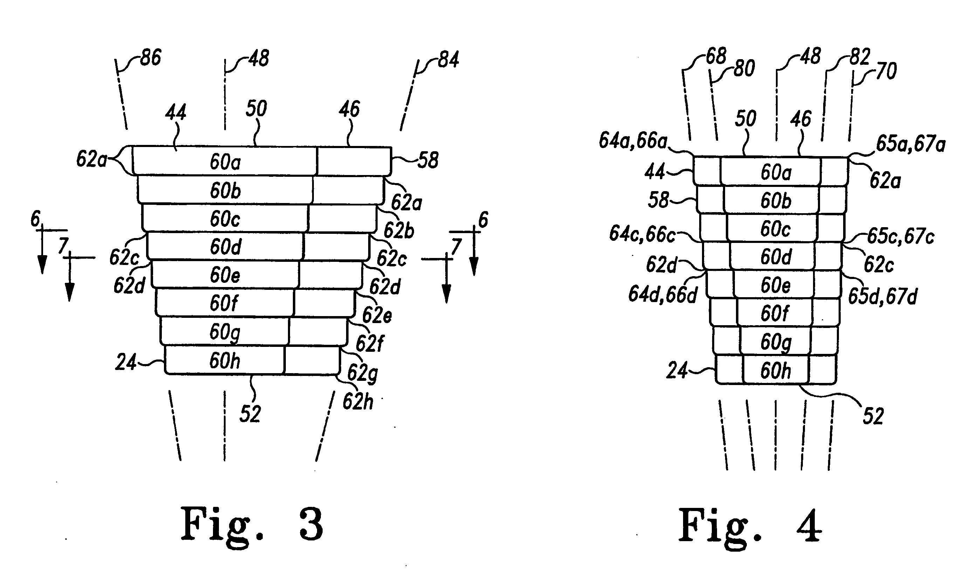

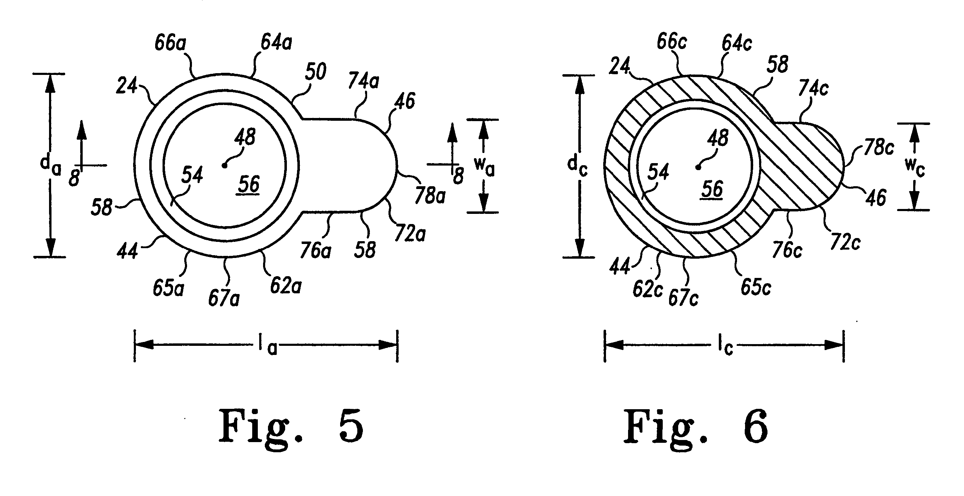

A first example of a prosthetic metaphyseal sleeve implant is shown in FIGS. 3 to 8. The first sleeve 24 is designed for use in a bone wherein the condition of the bone requires additional support or fixation on either the medial or lateral side of the metaphysis of the bone, but not on both sides; such a defect in the bone may be characterized as a non-centralized defect. The sleeve 24 comprises a body with a central portion 44 and a side wing portion 46. The central portion 44 has a central longitudinal axis 48 that defines a first end 50 and a second end 52. As shown in FIGS. 5 to 8, an inner wall 54 in the central portion 44 defines a channel 56 along the central longitudinal axis 48 of the central portion 44. The channel 56 tapers between the first and second ends. The tapered channel 56 may have any suitable taper angle for frictional locking with another element; for example, the tapered channel 56 may define a Morse taper. The channel 56 is designed to receive the tapered surface of the adapter 16 on the femoral side or the tapered surface of the stem portion 36 of the tibial tray 30 and to frictionally lock with one of these elements. Although it may be desirable to provide a set of sleeves 24 that can be used on either the tibial or the femoral side, separate sleeves may be used on the femoral side and on the tibial side, respectively; the shape of the channels 56 in such elements may vary to frictionally engage the adapter 16 if the sleeve is a femoral metaphyseal sleeve or to frictionally engage the stem portion 36 of the tibial tray 30.

The body of the sleeve 24 has an outer surface 58 that includes a plurality of adjacent terraces or steps. In the embodiment shown in FIGS. 3 to 8, the sleeve has eight terraces or steps, designated 60a-60h in FIGS. 3 and 4. As shown in FIG. 3, each terrace 60a-60h has an outer edge 62a-62h around its perimeter. The first terrace 60a has two outer edges of similar shape, one edge 62a being at the end 50 of the sleeve 24 and the edge, also designated 60a in FIG. 3, at the juncture of the first terrace 60a and the second terrace 60b. As shown in FIGS. 3 and 4, the outer edges 62a-62h of the terraces 60a-60h lie in planes that substantially perpendicular to the central longitudinal axis 48 of the central portion 44 of the body.

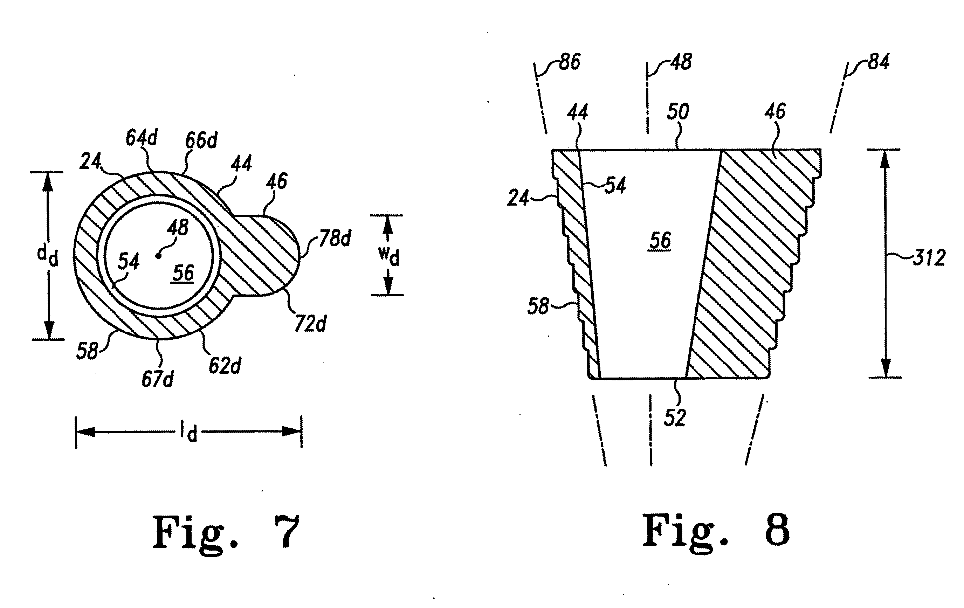

The shapes of the outer edges of representative terraces 60a, 60c and 60d are shown in FIGS. 5 to 7 at 62a, 62c and 62d. The outer edge 62a, 62c, 62d of each terrace 60a, 60c, 60d at the central portion 44 of the body comprises a pair of diametrically-opposed curved portions 64a, 64c, 64d, 65a, 65c, 65d having the same radius of curvature. The centre of curvature of each of the diametrically-opposed curved portions 64a, 64c, 64d, 65a, 65c, 65d is along the central longitudinal axis 48 of the body. The diametrically-opposed curved portions 64a, 64c, 64d, 65a, 65c, 65d define vertices 66a, 66c, 66d, 67a, 67c, 67d of the outer edges 62a, 62c, 62d at the central portion 44 of the body. Lines tangent to the vertices 66a, 66c, 66d, 67a, 67c, 67d of the outer edges 62a, 62c, 62d of the central portion 44 of the body are shown in FIG. 4 at 68 and 70. These tangent lines 68, 70 each define an angle of about 2 to 4°. The angle may vary depending on whether the sleeve is intended for use on the femoral or tibial side: for a femoral metaphyseal sleeve, for example, the angle may be about 2°; for a tibial metaphyseal sleeve, for example, the angle may be about 4°. Alternatively, a universal sleeve could be provided usable on either the tibial or femoral side. Thus, the diameter of each terrace, that is, the transverse dimension between opposite vertices, decreases between the first end 50 and the second end 52; for example, the diameter dₐ shown in FIG. 5 for the centre portion first terrace 60a is greater than the diameter d_{c} shown in FIG. 6 for the centre portion of terrace 60c that is closer to the second end 52, and the diameter d_{c} for the centre portion of terrace 60c is greater than the diameter d_{d} shown in FIG. 7 for the centre portion of terrace 60d that is still closer to the second end 52 of the sleeve.

The outer edge 62 of each terrace 60 at the side wing portion 46 of the body comprises a curved portion 72 joined to the diametrically-opposed curved portions 64 of the outer edge 62 at the centre portion 44. Examples are shown in FIGS. 5 to 7 for terraces 60a, 60c and 60d. As shown in FIGS. 5 and 6, the outer edges of the terraces of the side wing portion 46 may include a pair of spaced opposed straight lines 74a, 74c, 76a, 76c connected to the diametrically-opposed curved portions 64a, 64c, 65a, 65c and to the curved end segments 72a, 72c. Thus, the curved end segments 72a, 72c may be spaced from the diametrically-opposed curved portions 64a, 64c, 65a, 65c, of the outer edges of the central portion 44. As shown in FIG. 7, the curved segments 72d of the outer edge 62d may be directly connected to the segments 64d, 65d of the outer edge 62d at the centre portion 44, with no intervening straight segments. Each curved segment 72 of the outer edge 62 of each terrace 60 has a vertex 78a, 78c, 78d.

Lines tangent to the juncture of side wing portion 46 and the central portion 44 of the body of the sleeve 24 are shown in FIG. 4 at 80 and 82. These tangent lines 80, 82 each define an angle of about 2 to 4° with the plane of the central longitudinal axis 48.. The angle may vary depending on whether the sleeve is intended for use on the femoral or tibial side: for a femoral metaphyseal sleeve, for example, the angle may be about 2 °; for a tibial metaphyseal sleeve, for example, the angle may be about 4°. Alternatively, a universal sleeve could be provided usable on either the tibial or femoral side. Thus, the width of the side portion of each terrace, that is, the maximum transverse dimension of the side portion of each terrace, decreases between the first end 50 and the second end 52; for example, width wₐ shown in FIG. 5 for the centre portion first terrace 60a is greater than width w_{c} shown in FIG. 6 for the centre portion of terrace 60c that is closer to the second end 52 and width w_{c} of the centre portion of terrace 60c is greater than width wd shown in FIG. 7 for the centre portion of terrace 60d that is still closer to the second end 52 of the sleeve. As can also be seen in FIG. 4, the width of the side portion 46 of each terrace is less than the width of the centre portion 44 of each terrace 60. Each terrace 60, including its outer edge 62, is thus indented at the junctions of the diametrically-opposed curved portions 64a, 64c, 64d, 65a, 65c, 65d of the outer edge 62 at the centre portion 44 and the segments of the outer edge at the side wing portion 46.

The overall lengths of each terrace 60 also decrease between the first end 50 and the second end 52. As shown in FIG. 3, a line tangent with the vertices 78 of the side portions 46, shown at 84 in FIG. 3, may define a variety of angles with the plane of the central longitudinal axis 48; the angle would depend, for example, on the size of the implant. A line tangent with a point on each outer edge of each terrace opposite to the line 84, shown at 86 in FIG. 3, defines an angle of about 2 to 4° with the plane of the central longitudinal axis 48. The angle may vary depending on whether the sleeve is intended for use on the femoral or tibial side: for a femoral metaphyseal sleeve, for example, the angle may be about 2°; for a tibial metaphyseal sleeve, for example, the angle may be about 4°. Alternatively, a universal sleeve could be provided usable on either the tibial or femoral side. Thus, the length, that is the maximum transverse dimension of each terrace, decreases between the first end 50 and the second end 52; for example, width lₐ shown in FIG. 5 for the centre portion first terrace 60a is greater than length l_{c} shown in FIG. 6 for the centre portion of terrace 60c that is closer to the second end 52 and width w_{c} of the centre portion of terrace 60c is greater than width w_{d} shown in FIG. 7 for the centre portion of terrace 60d that is still closer to the second end 52 of the sleeve.

The sleeve 24 shown in FIGS. 3 to 8 is asymmetric about one transverse plane through the central longitudinal axis 48 and symmetric about a perpendicular plane through the central longitudinal axis 48. It should be understood that the above description of the features of sleeve 24 also applies to the single-winged sleeve 38 of FIG. 2.

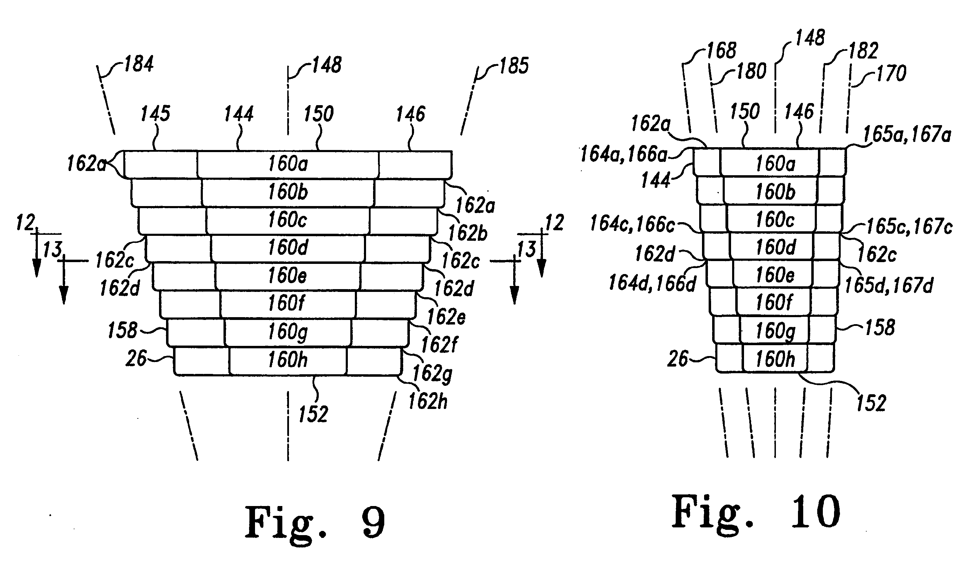

In contrast, the second sleeve 26 of FIGS. 9 to 13 is symmetric about two perpendicular planes through the central longitudinal axis 148 of the sleeve. The second sleeve 26 is designed for use in a bone wherein the condition of the bone requires additional support or fixation on both the medial and lateral sides of the metaphysis of the bone.

The second sleeve 24 comprises a body with a central portion 144, a first side wing portion 145 and a second side wing portion 146. The central portion 144 has a central longitudinal axis 148 that defines a first end 150 and a second end 152. As shown in FIGS. 5-8, an inner wall 154 in the central portion 144 defines a channel 156 along the central longitudinal axis 148 of the central portion 144. The channel 156 tapers between the first and second ends. The tapered channel 156 may have any suitable taper angle for frictional locking with another element; for example, the tapered channel 156 may define a Morse taper. The channel 156 is designed to receive the tapered surface of the adapter 16 on the femoral side or the tapered surface of the stem portion 36 of the tibial tray 30 and to frictionally lock with one of these elements. Although it may be desirable to provide a set of sleeves 26 that can be used on either the tibial or the femoral side, separate sleeves may be used on the femoral and tibial sides; the shape of the channels 156 in such elements may vary to frictionally engage the adapter 16 if the sleeve is a femoral metaphyseal sleeve or to frictionally engage the stem portion 36 of the tibial tray 30.

The body of the sleeve 26 has an outer surface 158 that includes a plurality of adjacent terraces or steps. In the embodiment shown in FIGS. 9 to 14, the sleeve 26 has eight terraces or steps, designated 160a-160h in FIGS. 9 and 10. As shown in FIG. 9, each terrace 160a-160h has an outer edge 162a-162h around its perimeter. The first terrace 160a has two outer edges of similar shape, one edge 162a being at the end 150 of the sleeve 26 and the edge, also designated 60a in FIG. 3, at the juncture of the first terrace 160a and the second terrace 160b. As shown in FIGS. 9 and 10, the outer edges 162a-162h of the terraces 160a-160h lie in planes that are substantially perpendicular to the central longitudinal axis 148 of the central portion 144 of the body.

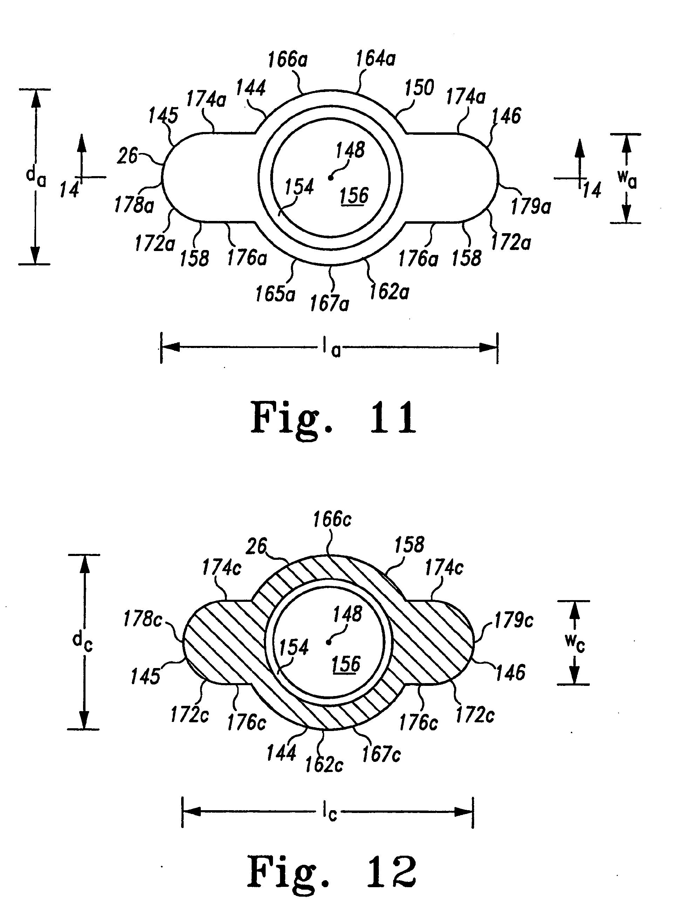

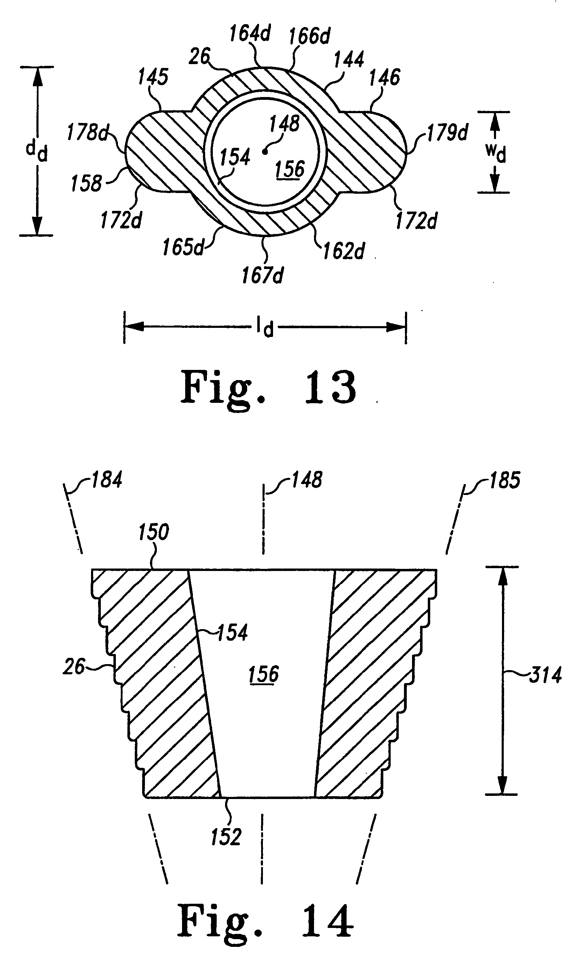

The shapes of the outer edges of representative terraces 160a, 160c and 160d are shown in FIGS. 11 to 13 at 162a, 162c and 162d. The outer edge 162a, 162c, 162d of each terrace 160a, 160c, 160d at the central portion 144 of the body comprises a pair of diametrically-opposed curved portions 164a, 164c, 164d, 165a, 165c, 165d having the same radius of curvature. The centre of curvature of each of the diametrically-opposed curved portions 164a, 164c, 164d, 165a, 165c, 165d is along the central longitudinal axis 148 of the body. The diametrically-opposed curved portions 164a, 164c, 164d, 165a, 165c, 165d define vertices 166a, 166c, 166d, 167a, 167c, 167d of the outer edges 162a, 162c, 162d at the central portion 144 of the body. Lines tangent to the vertices 166a, 166c, 166d, 167a, 167c, 167d of the outer edges 162a, 162c, 162d of the central portion 144 of the body are shown in FIG. 10 at 168 and 170. These tangent lines 168, 170 each define an angle of about 2 to 4 °. The angle may vary depending on whether the sleeve is intended for use on the femoral or tibial side: for a femoral metaphyseal sleeve, for example, the angle may be about 2°; for a tibial metaphyseal sleeve, for example, the angle may be about 4°. Alternatively, a universal sleeve could be provided usable on either the tibial or femoral side. Thus, the diameter of each terrace, that is, the transverse dimension between opposite vertices, decreases between the first end 150 and the second end 152; for example, diameter dₐ shown in FIG. 11 for the centre portion first terrace 160a is greater than diameter d_{c} shown in FIG. 12 for the centre portion of terrace 160c that is closer to the second end 152 and diameter d_{c} of the centre portion of terrace 160c is greater than diameter d_{d} shown in FIG. 13 for the centre portion of terrace 160d that is still closer to the second end 152 of the sleeve 26.

As can also be seen in FIGS. 5 to 7, the overall length "1" of each terrace 60 is the largest dimension of each terrace 60. The length of each terrace "l" is greater than its diameter "d": lₐ>dₐ; l_{c}>d_{c}; and l_{d}>d_{d}.

The outer edge 162 of each terrace 160 at each side wing portion 145, 146 of the body comprises a curved portion 172 joined to the diametrically-opposed curved portions 164, 165 of the outer edge 162 at the centre portion 144. Examples are shown in FIGS. 11 to 13 for terraces 160a, 160c and 160d. As shown in FIGS. 11 and 12, the outer edges of the terraces of the side wing portions 145, 146 may each include a pair of spaced opposed straight lines 174a, 174c, 176a, 176c connected to the diametrically-opposed curved portions 164a, 164c, 165a, 165c and to the curved end segments 172a, 172c. Thus, the curved end segments 172a, 172c may be spaced from the diametrically-opposed curved portions 164a, 164c, 165a, 165c, of the outer edges of the central portion 144. As shown in FIG. 7, the curved segments 172d of the outer edge 162d may be directly connected to the segments 164d, 165d of the outer edge 162d at the centre portion 144, with no intervening straight segments. Each curved segment 172 of the outer edge 162 of each terrace 160 has a vertex 178a, 178c, 178d.

Lines tangential to the junctures of the first side wing portion 145 and the central portion 144 of the body of the sleeve are shown in FIG. 10 at 180 and 182. These tangent lines 180, 182 each define an angle of about 2 to 4° with the plane of the central longitudinal axis 148. The second side wing portion 146 is similarly shaped. The angle may vary depending on whether the sleeve is intended for use on the femoral or tibial side: for a femoral metaphyseal sleeve, for example, the angle may be about 2°; for a tibial metaphyseal sleeve, for example, the angle may be about 4°. Alternatively, a universal sleeve could be provided usable on either the tibial or femoral side. Thus, the widths of the side portions 145, 146 of each terrace, that is, the maximum transverse dimensions of the side wing portions of each terrace, decreases between the first end 150 and the second end 152; for example, width wₐ shown in FIG. 11 for the centre portion first terrace 160a is greater than width w_{c} shown in FIG. 12 for the centre portion of terrace 160c that is closer to the second end 152 and width w_{c} of the centre portion of terrace 160c is greater than width w_{d} shown in FIG. 13 for the centre portion of terrace 160d that is still closer to the second end 152 of the sleeve. As can also be seen in FIGS. 11 to 13, the width of the side portions 145, 146 of each terrace is less than the width of the centre portion 144 of each terrace 160. Each terrace 160, including its outer edge 162, is thus indented at the junctions of the diametrically-opposed curved portions 164a, 164c, 164d, 165a, 165c, 165d of the outer edge 162 at the centre portion 144 and the segments of the outer edge at the side wing portion 146.

The overall lengths of each terrace 160 also decrease between the first end 150 and the second end 152. As shown in FIG. 9, lines tangent with the vertices 178, 179 (shown in FIGS. 11 to 13) of the side portions 145, 146, shown at 184 and 185 in FIG. 9, may define a variety of angles with the plane of the central longitudinal axis 148; the angle would depend, for example, on the size of the implant. Thus, the length, that is the maximum transverse dimension of each terrace 160, decreases between the first end 150 and the second end 152; for example, length lₐ shown in FIG. 11 for the centre portion of the first terrace 160a is greater than length l_{c} shown in FIG. 12 for the centre portion of terrace 160c that is closer to the second end 152 and length l_{c} of the centre portion of terrace 160c is greater than length l_{d} shown in FIG. 13 for the centre portion of terrace 160d that is still closer to the second end 152 of the sleeve. As can also be seen in FIGS. 11 to 13, the overall length of each terrace 160 is the largest dimension of each terrace 160.

It should be understood that the above description of sleeve 26 also applies to the double-winged metaphyseal sleeve 40 of FIG. 2.

It should be understood that a typical modular knee prosthesis kit would include several sizes of metaphyseal sleeves, and may include several sizes of each of the sleeves 24, 26, 38, 40. All of such sleeves may have features like those described above. In addition, the sleeves of the kit may have additional features to enhance fixation. For example, the sleeves might be provided with a porous coating, for example, as disclosed in US-A-2005/0107883.

In the embodiments which are described above, the terraces 60, 160 of the sleeves 24, 26 provide parallel planar surfaces perpendicular to the axis 48, 148. The distances between these parallel planar surfaces define the height of each terrace 60, 160 or step. In the described embodiments, the heights of the terraces are all substantially the same, although it should be understood that any given sleeve could have terraces of different heights, and different sizes of sleeves could have terraces of the same or different heights.

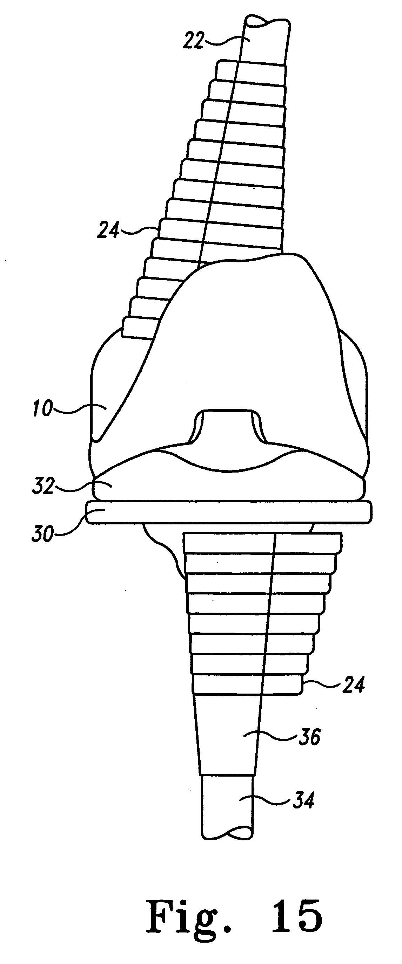

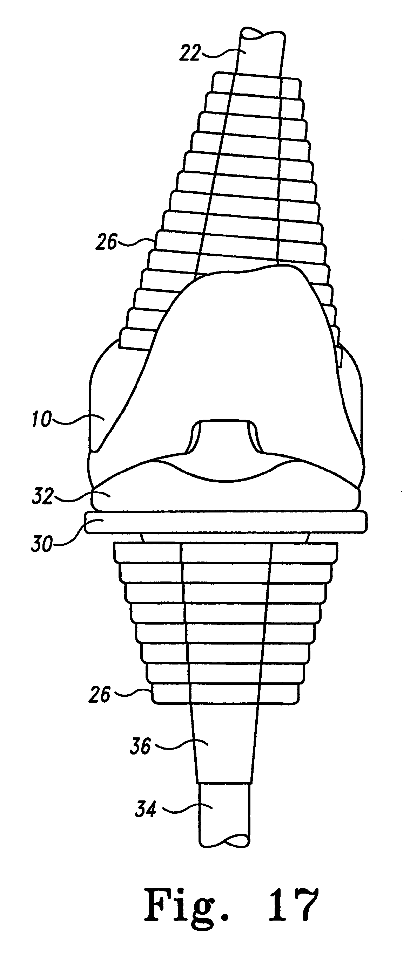

As discussed above, the channels 56, 156 of the sleeves 24, 26 are tapered to allow for frictional connection to components of the femoral side and tibial side. Examples of assemblies of the modular knee prostheses components of FIGS. 1 and 2 are shown in FIGS. 15 to 17. Although not shown, the combined assemblies could also include a single-winged metaphyseal sleeve 24 on one side of the joint motion surface and a double-winged metaphyseal sleeve 26 on the other side of the joint motion surface, or a metaphyseal sleeve of either type 24, 26 could be used on only one side of the joint motion surface. Use of these variations will depend on the condition of the patient's bone. Advantageously, with the modular system of the present invention, the implants can be substantially customized intraoperatively to fit the needs of the individual patient.

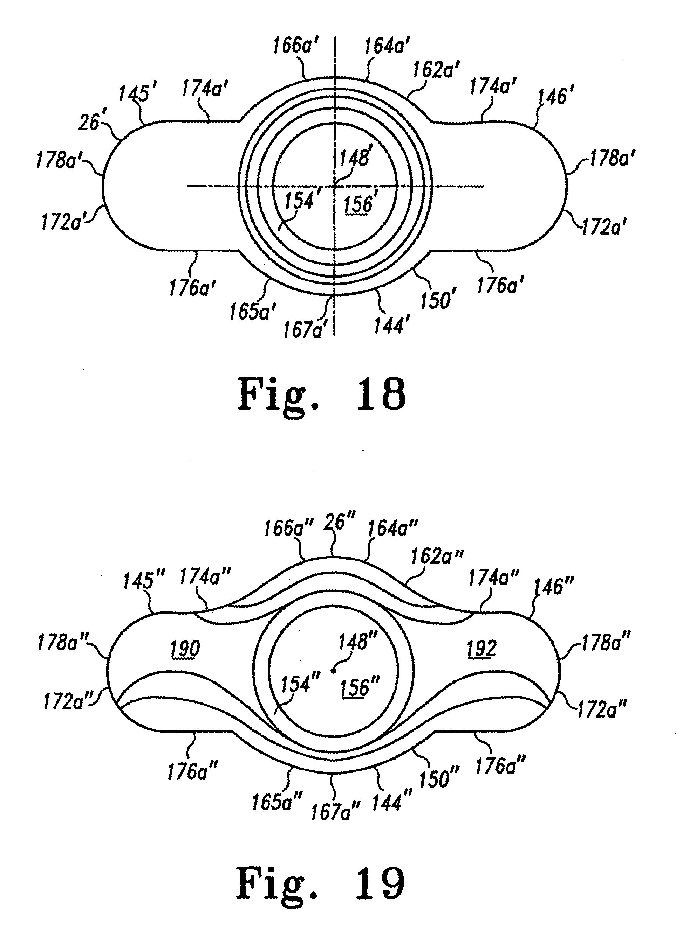

It should be understood that although the curved segments 64, 65, 72, 164, 165, 172 of the metaphyseal sleeves shown in FIGS. 5 to 7 and 11 to 13 comprise circular arcs, the curved segments could have other shapes. For example, the diametrically-opposed curved segments could be elliptical in shape, such as shown in FIG. 18. In the embodiment of FIG. 18, the same reference numerals are used for elements similar to those of the embodiment of FIGS. 11 to 13, followed by the prime symbol. Thus, the metaphyseal sleeve is designated 26'. It should also be understood that not all segments of the outer edges of all the terraces would need to have the same shape. For example, the outer edges of the terraces near end 150' may have elliptical-shaped diametrically-opposed curved segments 164', 165' but the outer edges of the terraces near end 152' may have diametrically-opposed curved segments 164, 165 that define circular arcs. Similar variations are possible for the curved edges 172 of the side wing portions 145, 146.

If the sleeves on the tibial side are to be used with a tibial tray of the type having keels, then it may be desirable to include reliefs in the terraces at the end nearest the joint motion surface to accommodate the keels. Suitable reliefs allowing for rotational adjustment of the sleeve with respect to the tibial tray are disclosed in US-7291174, and may similarly be employed with the sleeves of the present invention. An example of a double-winged metaphyseal sleeve with such reliefs is shown in FIG. 19. In the embodiment of FIG. 19, the same reference numerals are used for elements similar to those of the embodiment of FIGS. 11 to 13, followed by the double prime symbol. Thus, the metaphyseal sleeve is designated 26". The reliefs are designated 190 and 192 in FIG. 19. These reliefs 190, 192 would extend down through the first few terraces nearest end 150" to accommodate the keels on the tray. Keels are shown in FIG. 2 at 194, 196.

The complete kit for the knee prosthesis system will also include instrumentation to be used in preparing the bone to receive the implants. The complete kit of the present invention will preferably include reamers and milling guides for use in preparing the bone to receive a metaphyseal sleeve of the types described above.

One of the reamers (not shown) may comprise a standard straight reamer for preparing a straight pilot hole into the intramedullary canal. An example of an appropriate reamer is disclosed in US-4790852. A typical instrument set would include several sizes of such straight reamers.

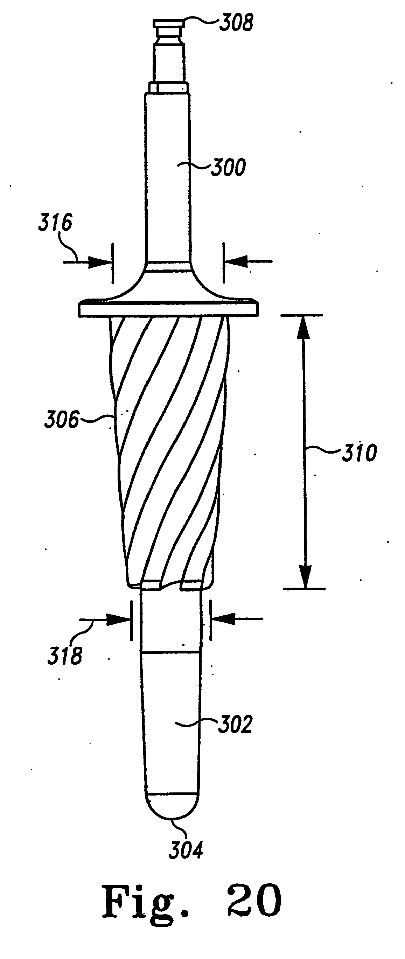

Examples of a second type of reamer are shown in FIGS. 20 to 21. The reamer 300 of FIG. 20 is used to prepare a conical cavity in the bone, aligned with the intramedullary canal, to receive the central portion 44, 144 of one of the metaphyseal sleeves 24, 26, 26', 26". The conical reamer 300 includes a pilot shaft 302 at its distal end 304 for aligning the conical reamer 300 with the intramedullary canal or the pilot hole formed by the straight reamer. The reamer also includes a fluted cutting portion 306 between the pilot shaft 302 and the proximal end 308 of the conical reamer 300. The fluted cutting portion 306 defines a tapered cutting envelope having a length L. The fluted cutting portion 306 tapers from a maximum diameter toward the proximal end 308 to a minimum diameter at the juncture with the pilot shaft 302. The length of the fluted cutting portion 306, shown at 310 in FIG. 20, corresponds with the length of a selected metaphyseal sleeve, such as shown at 312 and 314 in FIGS. 8 and 14. The maximum diameter of the fluted cutting portion is shown at 316 in FIG. 20 and the minimum diameter is shown at 318 in FIG. 20. The maximum diameter 316 of the fluted cutting portion corresponds with the maximum diameter of the central portion 44, 144, 144', 144" of the selected metaphyseal sleeve 24, 26, 26', 26", such as diameter dₐ shown in FIGS. 5 and 11. The minimum diameter 318 of the fluted cutting portion corresponds with the minimum diameter of the central portion 44, 144, 144', 144" of the selected metaphyseal sleeve 24, 26, 26', 26". The taper angle for the fluted cutting portion 306 corresponds with the taper angles defined by the tangent lines 68, 70, 168, 170 shown in FIGS. 4 and 10. The proximal end 308 of the conical reamer 300 provides a fitting for connecting the conical reamer to a handle or power source for rotating the conical reamer to prepare the conical cavity.

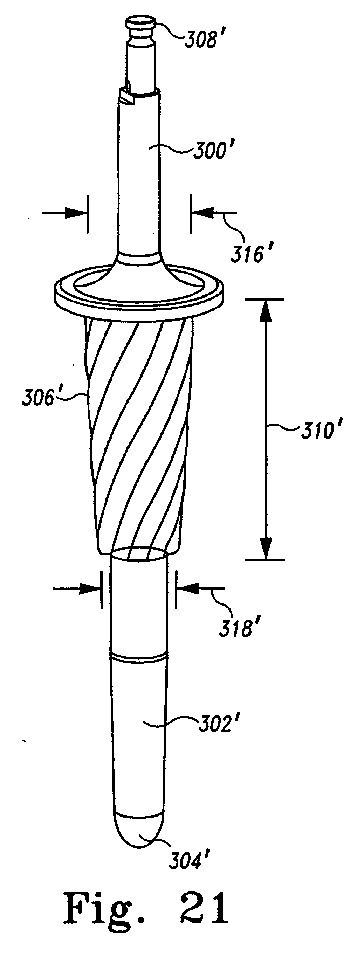

FIG. 21 shows a second example of a conical reamer with a longer pilot shaft than in the embodiment of FIG. 20. In the embodiment of FIG. 21, the same reference numerals are used for elements similar to those of the embodiment of FIG. 20, followed by the prime symbol. Thus, the conical reamer is designated 300'.

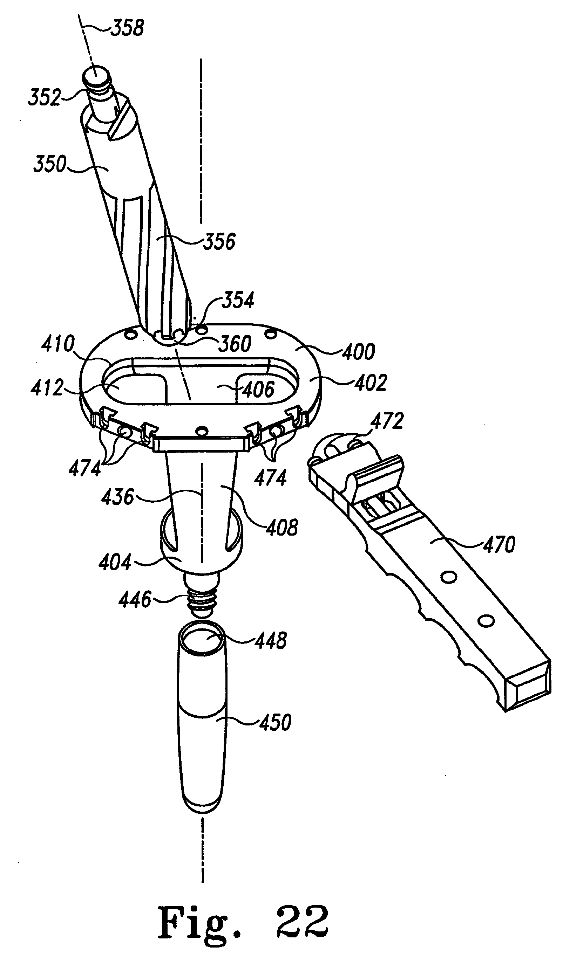

Examples of other instruments that would be included in the kit are shown in FIGS. 22 to 28. These other instruments include a straight reamer 350. The straight reamer 350 may be similar to standard reamers with a proximal end 352, a distal end 354 and a fluted cutting portion 356 between the two ends. The reamer has a central longitudinal axis 358.

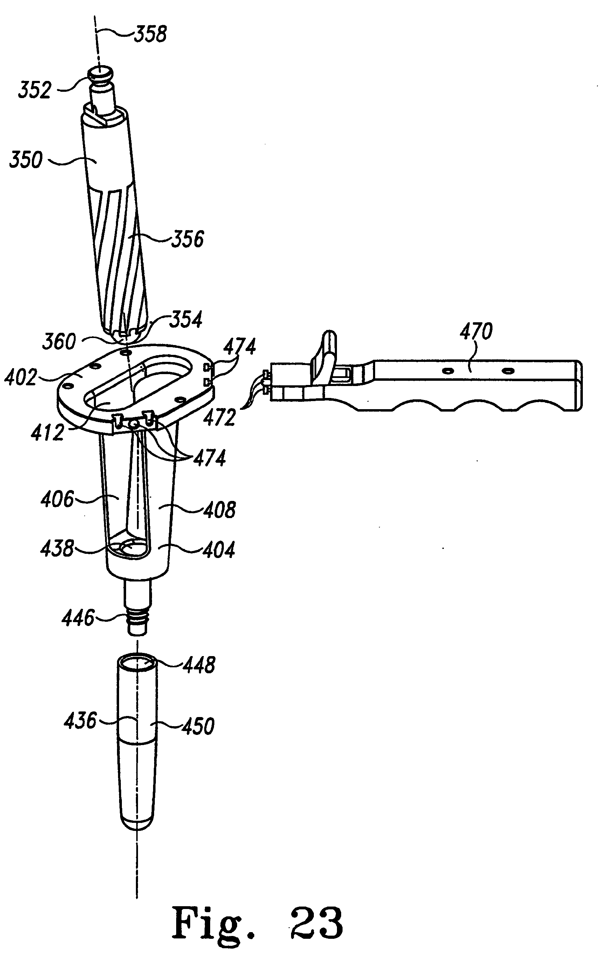

The proximal end 352 of the straight reamer 350 provides a fitting for connecting the reamer 350 to a handle or power source for rotating the reamer to prepare the bone cavity. The fluted cutting portion 356 defines a substantially cylindrical cutting envelope. The distal end 354 of the straight reamer 350 comprises a convex spherical surface 360, best seen in FIG. 23.

Preferably, the kit includes a milling guide. An example of a milling guide that may be used in preparing bone to receive metaphyseal sleeves such as double winged metaphyseal sleeve 26 is shown in FIGS. 22 to 28. The milling guide 400 comprises a template member 402, a base 404 and a pair of side walls 406, 408 connecting the template member 402 and the base 404. The template member 402 has an inner surface 410 defining an elongate milling guide opening 412. The elongate milling guide opening 412 is shaped to correspond with the shape defined by the two side wing portions 145, 145', 145", 146, 146', 146" of the double winged metaphyseal sleeve 26, 26', 26".

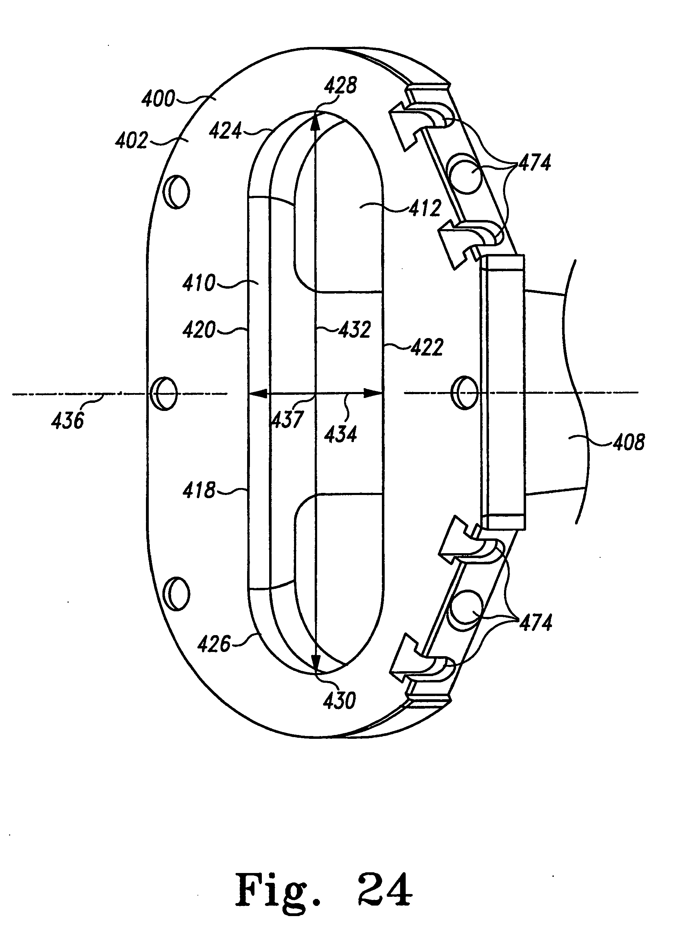

As shown in FIG. 24, the elongate milling guide opening 412 has an edge 418 including two parallel straight portions 420, 422 and two curved end portions 424, 426. The two curved end portions 424, 426 connect the two parallel straight portions 420, 422. Each curved end portion 424, 426 has a vertex 428, 430. The maximum first transverse dimension 432 of the elongate milling guide opening 412 is at the vertices 428, 430 of the curved end portions 424, 426. The second transverse dimension 434 of the elongate milling guide opening 412 is the distance between the two parallel straight portions 420, 422, and is less than the first transverse dimension 432. A central longitudinal axis 436 extends from the centre 437 of the elongate milling guide opening 412 and through the centre of the spherical depression of the base. The vertices 428, 430 of the curved end portions 424, 426 of the elongate milling guide opening 412 and the central longitudinal axis 436 of the milling guide lie in a plane.

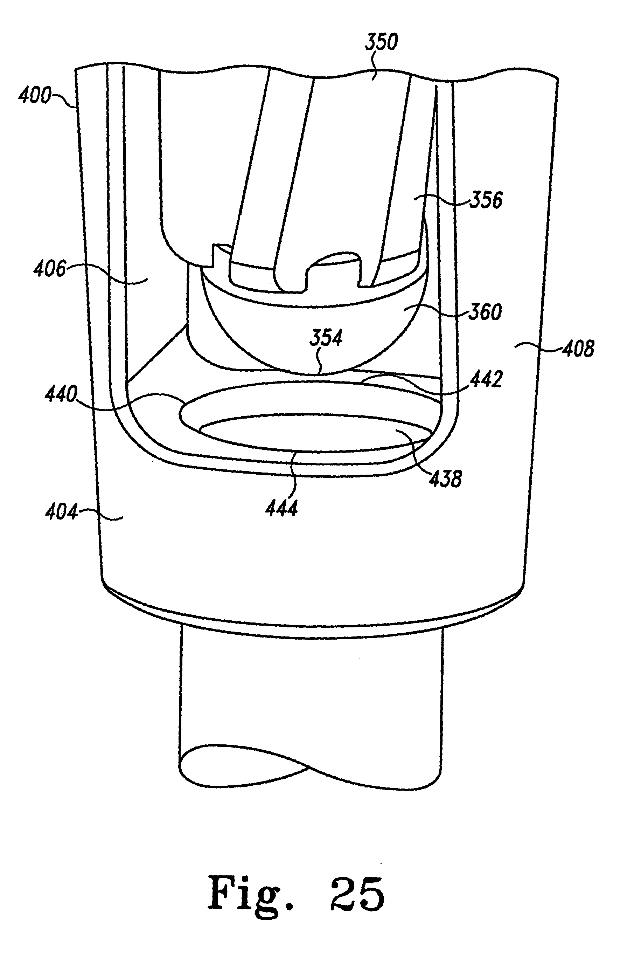

The base 404 is spaced from the template member 402 and includes a concave spherical depression 438 aligned with the milling guide opening 412 along the central longitudinal axis 436. As shown in FIG. 25, the concave spherical depression 438 of the base 404 of the milling guide 400 has a perimeter 440 and opposed vertices 442, 444 lying in the same plane as the vertices 428, 430 of the curved end portions 424, 426 of the elongate milling guide opening 412 and the longitudinal axis 436 of the milling guide. A line from each vertex 428, 430 of the of the curved end portions 424, 426 of the elongate milling guide opening 412 to the nearest vertex 442, 444 of the perimeter 440 of the concave spherical depression 438 of the base 404 defines an acute angle with the longitudinal axis 436 of the milling guide 400.

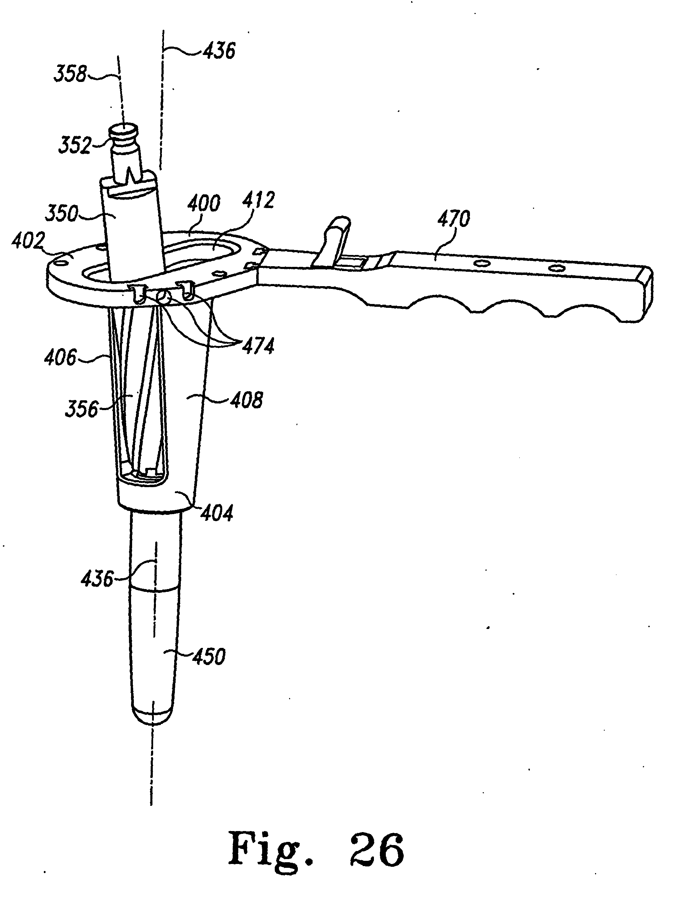

The concave spherical depression 438 is sized and shaped to receive the convex spherical surface 360 of the straight reamer 350 and allow the straight reamer 350 to be pivoted about the convex spherical surface 360 as shown in FIGS. 26 to 28. As the straight reamer 350 is so pivoted in depression 438 about convex spherical surface 360, the fluted cutting portion 356 of the reamer 350 cuts a concavity in the bone corresponding with a shape defined by the envelope of the side wing portions 145, 145', 145", 146, 146', 146" of the metaphyseal sleeve 26, 26', 26".

As shown in FIGS. 22 to 23, the base 404 of the milling guide 400 includes a threaded male member 446 extending distally away from the base 404 along the central longitudinal axis 436. The threaded male member 446 is received in a female threaded bore 448 in a stem trial 450. When assembled, as shown in FIGS. 26 to 28, the stem trial 450 extends distally from the base 404 along the central longitudinal axis 436 of the milling guide 400. The stem trial 450 is sized and shaped to be received in the bore created in the area of the intramedullary canal by the first straight reamer. Thus, the concavity created by the reamer 350 will be centred on and aligned with the intramedullary canal.

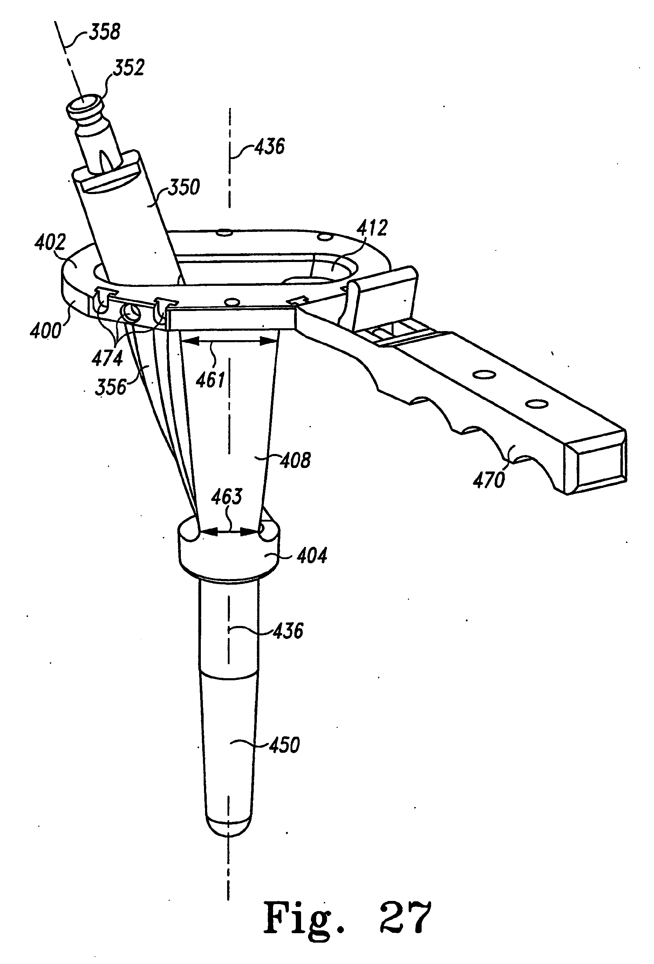

The side walls 406, 408 of the milling guide 400 taper from a maximum width at the juncture with the template member 402 to a minimum width at the juncture with the base 404. The maximum width is shown at 461 and the minimum width is shown at 463 in FIG. 27. The overall length of the milling guide between the distal side of the template member 402 and the concave spherical depression 438 is shown at 465 in FIG. 27. These dimensions 461, 463 and 465 correspond generally with the dimensions 310, 310', 316, 316', 318, 318' (see FIGS. 20 and 21) of the fluted cutting portion 306, 306' of the conical reamer 300, 300'. Thus, the side walls 406, 408 and base 404 of the milling guide 400 should fit within the conical concavity created by the conical reamer.

The instrument set may also include a handle 470, shown in FIGS. 22, 23 and 26 to 28. The handle 470 and the template member 402 may include complementary mounting members and apertures, shown generally at 472 and 474 in FIGS. 22 and 23. In the described embodiment, two sets of apertures 474 are provided on the template member 402 to allow the handle to be attached to either the medial or lateral side of the template member. The handle might include complementary mounting members and apertures having features which are disclosed in US-5733290.

It will be appreciated that, like the implant components of the knee prosthesis kit, the above-described instruments, including the reamers and the milling guide, will typically be provided in a variety of sizes corresponding with the sizes of the metaphyseal sleeve implant components.

In use, the surgeon will typically make initial bone cuts to the tibia and femur and determine whether the condition of the patient's bone would make it desirable to use a metaphyseal sleeve component. If so, the surgeon will determine whether the bone condition makes a single-wing metaphyseal sleeve or a double-wing metaphyseal sleeve is desirable and determine the appropriate size of sleeve.

On the tibial side, the surgeon would first use a straight reamer to prepare a pilot hole aligned with and extending into the intramedullary canal of the bone. Next, based on the diameter of the central portion 44, 144 of the selected metaphyseal sleeve 24, 26, 26', 26", the surgeon selects an appropriate sized conical reamer 300. The pilot shaft 302 of the conical reamer 300 is inserted into the pilot hole prepared with the straight reamer, and the conical reamer 300 is rotated and moved distally so that the fluted cutting portion 306 creates a conical concavity in the bone. This conical concavity will correspond in size and shape with the size and shape of the central portion 44, 144 of the metaphyseal sleeve 24, 26, 26', 26", and will be aligned with the intramedullary canal. The conical reamer is removed.

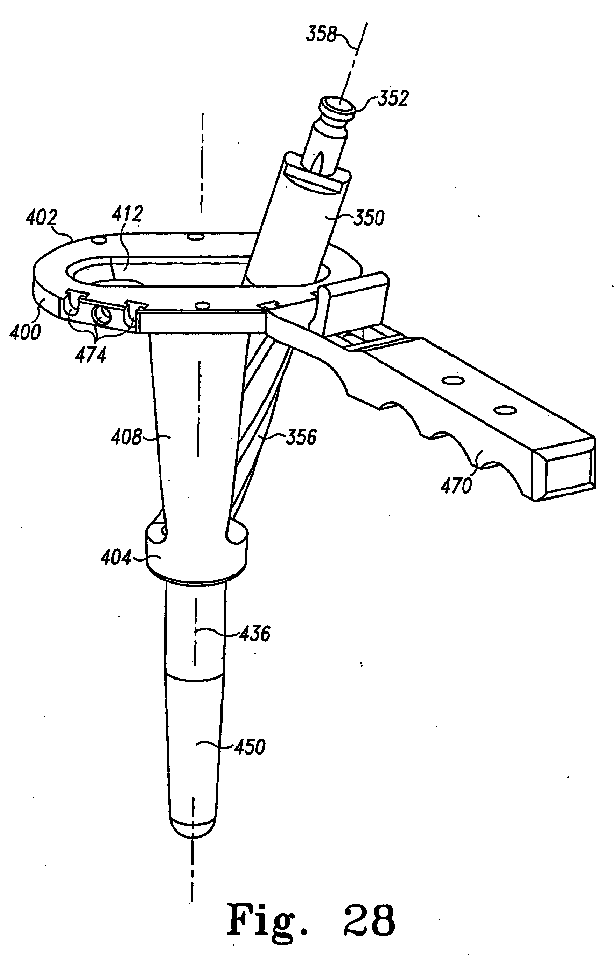

The surgeon would then assemble the appropriately-sized milling guide 400 with the appropriately sized stem trial 450. The stem trial portion 450 is inserted into the concavity created by the reaming steps and the milling guide 400 is moved distally so that the base 404 and side walls 406, 408 are received in the concavity. A straight reamer 350 is inserted into the milling guide opening 412 and moved distally until the convex spherical surface 360 is received in the concave spherical depression 438 of the base 404 of the milling guide 400. The straight reamer 350 is then rotated about its central longitudinal axis 358 while it is pivoted about the convex spherical surface 360 in the depression 438. FIGS. 27 and 28 show the straight reamer 350 pivoted as described above. If the defect is non-centralized and the surgeon has selected a single-wing metaphyseal sleeve, the surgeon would pivot the straight reamer 350 either medially or laterally. If the defect is centralized and the surgeon has selected a double-wing metaphyseal sleeve, the straight reamer 350 would be pivoted both medially and laterally. After the straight reamer 350 has been so rotated and pivoted and moved along the edge 418 of the milling guide opening 412, a concavity should be created that is sized and shaped to receive one of the metaphyseal implant components 24, 26, 26', 26", 38, 40.

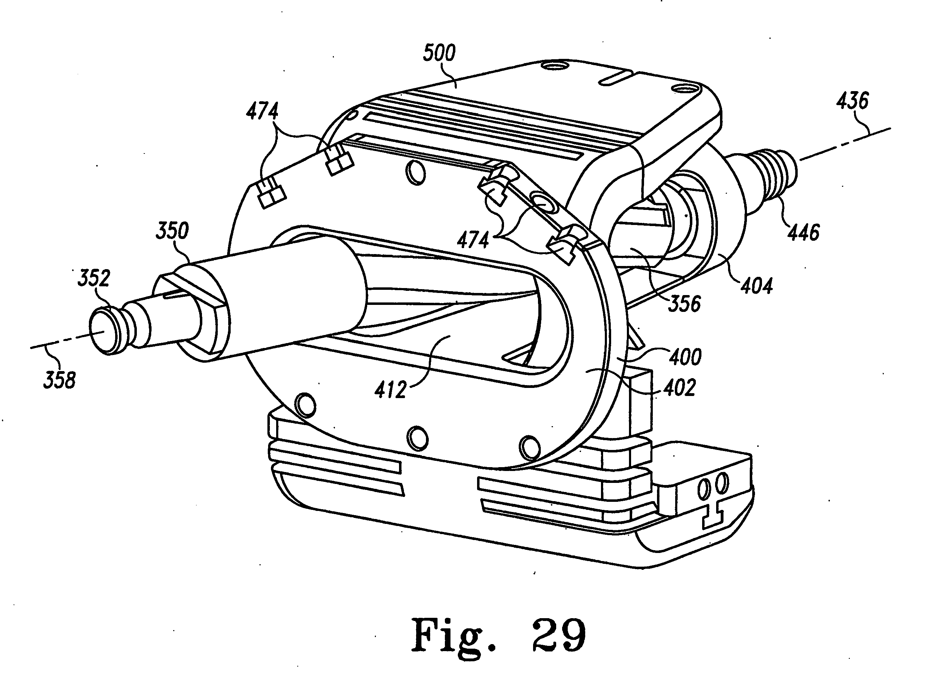

A similar procedure would be followed on the femoral side. As shown in FIG. 29, the milling guide 400 can be used while a separate femoral cutting block 500 is in place on the distal femur.

It should be understood that the same instruments and a similar procedure can be used when the metaphyseal implant is a single-winged sleeve such as sleeve 24. In such a case, instead of pivoting the straight reamer 350 along the entire length of the milling guide opening 412, the straight reamer 350 would only be pivoted along a portion of the length of the milling guide opening. Alternatively, a separate set of milling guides with milling guide openings sized and shaped to correspond with the size and shape of the proximal ends of the single winged sleeves 24 could be used.

Since the conical reamer and milling guide are positioned to be aligned with the intramedullary canal, the concavity created should be properly positioned regardless of whether the metaphyseal bone is harder in places or not. Accordingly, the metaphyseal implant should be properly positioned, rather than positioned too anterior or too posterior, avoiding flexion gaps and problematic joint balancing.

## Claims

1. A modular knee prosthesis kit comprising:
(a) a femoral component (10) with curved convex condylar surfaces (12, 14),
(b) a tibial component (30) with curved concave condylar surfaces to receive the curved convex condylar surfaces of the femoral component, the curved concave condylar surfaces of the tibial component and curved convex condylar surfaces of the femoral component defining a joint motion surface, and
(c) a sleeve (24, 38) comprising a body having a central longitudinal axis (48) which defines first and second ends (50, 52), a central portion (44), a side wing portion (46) extending from the central portion, and an inner wall (54) defining a channel (56) along the central longitudinal axis, the channel tapering between the first and second ends, in which the body has an outer surface (58) which includes a plurality of adjacent terraces (60a-60h), each terrace having an outer edge (62a-62h) around its perimeter, the outer edge of each terrace lying in a plane substantially perpendicular to the central longitudinal axis of the body,
in which the outer edge of each terrace at the central portion of the body comprises a pair of diametrically-opposed curved portions (64a, 64c, 64d, 65a, 65c, 65d) defining vertices of the outer edge at the central portion of the body, and the outer edge of each terrace at the side wing portion of the body is connected to the diametrically-opposed curved portions and has a curved end segment (72a, 72c) spaced from the diametrically-opposed curved portions of the central portion, the curved end segment defining a vertex (78) of the outer edge at the side wing portion of the body, each terrace having a first transverse dimension ("l") which is the largest dimension of the terrace, and a second transverse dimension ("d") between the vertices of the diametrically opposed curved portions of the outer edge at the central portion, the first transverse dimension of the terrace nearest the first end being greater than the first transverse dimension of the adjacent terrace and the second transverse dimension of the terrace nearest the first end being greater than the second transverse dimension of the adjacent terrace,
in which (i) the tibial component comprises a tibial tray (30) having a stem (36) and a tibial insert (32) carried by the tibial tray, and in which the sleeve defines a tibial sleeve, the tibial sleeve and the stem being sized and shaped so that the tibial sleeve is mountable on the stem with the stem extending through the channel of the sleeve and frictionally locked to the sleeve, or (ii) the kit includes a femoral adapter (16) mountable to the femoral component (10), and in which the sleeve defines a femoral sleeve, the femoral sleeve and the femoral adapter being sized and shaped so that the femoral sleeve is mountable on the adapter with the adapter extending through the channel of the sleeve and frictionally locked to the sleeve,
**characterised in that** the sleeve has a single side wing portion extending from the central portion so that the sleeve is asymmetric.

2. The kit of claim 1, in which the maximum transverse dimension of the sleeve (24) is at the first transverse dimension.

3. The kit of claim 1, in which the outer edge of each terrace (60a-60h) is indented at the junction of central portion (44) and the side wing portion (46).

4. The kit of claim 1, in which the sleeve is asymmetric about two perpendicular planes through the central longitudinal axis (48) of the body of the sleeve (24).

5. The kit of claim 4, which includes a second sleeve (26, 40), the second sleeve comprising:
a body having a central longitudinal axis (148) which defines first and second ends (150, 152), a central portion (144), a first side wing portion (145) , a second side wing portion (146) and an inner wall (154) defining a channel (156) along the central longitudinal axis, the channel tapering between the first and second ends,
said body having an outer surface (158) which includes a plurality of adjacent terraces (160a-160h), each terrace having an outer edge (162a-162h) around its perimeter, the outer edge of each terrace lying in a plane substantially perpendicular to the central longitudinal axis of the body,
the outer edge of each terrace at the central portion of the body comprising a pair of diametrically-opposed curved portions having the same radius of curvature, the centre of curvature of each of the diametrically-opposed curved portions (164a, 164c, 164d, 165a, 165c, 165d) being along the central longitudinal axis of the body, the diametrically-opposed curved portions defining vertices (166a, 166c, 166d, 167a, 167c, 167d) of the outer edge at the central portion of the body,
the outer edge of each terrace at the first side wing portion of the body comprising a pair of spaced opposed straight lines (174, 176) connected to the diametrically-opposed curved portions and having a curved end segment (172a, 172c) spaced from the diametrically-opposed curved portions of the central portion, the distance between the spaced opposed lines of the outer edge at the first side wing portion being less than the distance between the diametrically-opposed curved portions of the outer edge at the central portion of the body, the curved end segment defining a vertex (178) of the outer edge at the second side wing portion of the body,
the outer edge of each terrace at the second side wing portion of the body comprising a pair of spaced opposed straight lines (174, 176) connected to the diametrically-opposed curved portions of the central portion of the body and by a curved end segment (172a, 172c) spaced from the diametrically-opposed curved portions, the distance between the opposed lines being of the outer edge at the second side wing portion being less than the distance between the diametrically-opposed curved portions of the outer edge at the central portion of the body, the curved end segment defining a vertex (179) of the outer edge at the second side wing portion of the body,
each terrace having a first transverse dimension ("l") between the vertices of the outer edge at the first side wing portion and second side wing portion and a second transverse dimension ("d") between the vertices of the diametrically opposed curved portions of the outer edge at the central portion,
in which the first transverse dimension of the terrace nearest the first end is greater than the first transverse dimension of the adjacent terrace and the second transverse dimension of the terrace nearest the first end is greater than the second transverse dimension of the adjacent terrace, and
in which the sleeve is symmetric about two perpendicular planes through the central longitudinal axis of the body of the sleeve.

6. The kit of claim 1, which includes a milling guide (400) for use in forming a cavity to receive the sleeve, the milling guide comprising:
a template member (402) having an inner surface (410) defining an elongate milling guide opening, the elongate milling guide opening (412) having a first transverse dimension and a second transverse dimension, the first transverse dimension of the elongate milling guide opening being at least as great as the first transverse dimension of the terrace of the sleeve nearest the first end of the sleeve, the second transverse dimension being less than the first transverse dimension,
a base (404) spaced from the template member, the base including a spherical depression (438) aligned with the milling guide opening, and
a side wall (406, 408) connecting the template member and the base member.

7. The kit of claim 6, which includes a stem trial (450), the stem trial and the milling guide (400) having complementary features for connecting the stem trial to the base (404) of the milling guide.

8. The kit of claim 7, which includes a reamer (350) having a first end, a second end and a central longitudinal axis, the first end of the reamer comprising a convex spherical surface sized and shaped to be received in the spherical depression (438) of the base (404) of the milling guide (400), the reamer being sized and shaped to extend from the base of the milling guide and through the elongate milling guide opening (412).

9. The kit of claim 8, which includes a second reamer, the second reamer having a first end, a second end, a central longitudinal axis and a maximum diameter, in which the maximum diameter of the second reamer is greater than the second transverse dimension of the elongate milling guide opening.

10. The kit of claim 9, in which the central portion (44) of the sleeve (24, 38) has a maximum diameter and in which the maximum diameter of the second reamer is the same as the maximum diameter of the central portion of the sleeve.

11. The kit of claim 8, in which the spherical depression (438) of the base (404) is sized and shaped to limit translational movement of the convex spherical surface of the first end of the reamer to a distance less than the first transverse dimension of the elongate milling guide opening (412).

12. The kit of claim 6 in which the centre of the elongate milling guide opening (412) and the centre of the spherical depression (438) of the base (404) lie on a longitudinal axis.

## Patentansprüche

1. Basisausrüstung für eine modulare Knieprothese, die aufweist:
(a) eine Oberschenkelkomponente (10) mit gekrümmten konvexen Kondylenflächen (12, 14),
(b) eine Schienbeinkomponente (30) mit gekrümmten konkaven Kondylenflächen, um die gekrümmten konvexen Kondylenflächen der Oberschenkelkomponente aufzunehmen, wobei die gekrümmten konkaven Kondylenflächen der Schienbeinkomponente und die gekrümmten konvexen Kondylenflächen der Oberschenkelkomponente eine Gelenkbewegungsfläche definieren, und
(c) eine Hülse (24, 38), die einen Körper mit einer mittigen Längsachse (48), welche ein erstes und ein zweites Ende (50, 52) definiert, einen mittleren Bereich (44), einen Seitenflügelbereich (46), der sich von dem mittleren Bereich erstreckt, und eine innere Wand (54), die einen Kanal (46) entlang der mittigen Längsachse definiert, aufweist, wobei sich der Kanal zwischen dem ersten und dem zweiten Ende verjüngt, wobei der Körper eine Außenfläche (58) hat, die eine Vielzahl aneinander grenzender Terrassen (60a-60h) umfasst, wobei jede Terrasse eine Außenkante (62a-62h) um ihren Durchmesser hat, wobei die Außenkante jeder Terrasse in einer Ebene liegt, die im Wesentlichen senkrecht zu der mittigen Längsachse des Körpers ist,
wobei die Außenkante jeder Terrasse an dem mittleren Bereich des Körpers ein Paar einander diametral gegenüberstehender, gekrümmter Bereiche (64a, 64c, 64d, 65a, 65c, 65d) aufweist, die Scheitelpunkte der Außenkante an dem mittleren Bereich des Körpers definieren, und die Außenkante jeder Terrasse an den Seitenflügelbereich des Körpers mit den einander diametral gegenüberstehenden, gekrümmten Bereichen verbunden ist und ein gekrümmtes Endsegment (72a, 72c) hat, das von den einander diametral gegenüberstehenden, gekrümmten Bereichen des mittleren Bereiches beabstandet ist, wobei das gekrümmte Endsegment einen Scheitelpunkt (78) der Außenkante an den Seitenflügelbereich des Körpers definiert, wobei jede Terrasse eine erste Querabmessung ("l"), die die größte Abmessung der Terrasse darstellt, und eine zweite Querabmessung ("d") zwischen den Scheitelpunkten der einander diametral gegenüberstehenden, gekrümmten Bereiche der Außenkante an dem mittigen Bereich hat, wobei die erste Querabmessung der Terrasse, die dem ersten Ende am nächsten liegt, größer ist als die erste Querabmessung der angrenzenden Terrasse und die zweite Querabmessung der Terrasse, die dem ersten Ende am nächsten liegt, größer ist als die zweite Querabmessung der angrenzenden Terrasse,
wobei (i) die Schienbeinkomponente eine Schienbeinablage (30) mit einem Schaft (36) und eine Schienbeineinlage (32), die von der Schienbeinablage getragen wird, aufweist und wobei die Hülse eine Schienbeinhülse definiert, wobei die Schienbeinhülse und der Schaft so bemessen und geformt sind, dass die Schienbeinhülse auf dem Schaft anbringbar ist, wobei sich der Schaft durch den Kanal der Hülse erstreckt und durch Reibschluss an der Hülse gehalten ist, oder (ii) die Basisausrüstung einen Oberschenkeladapter (16) umfasst, der an der Oberschenkelkomponente (10) anbringbar ist, und wobei die Hülse eine Oberschenkelhülse definiert, wobei die Oberschenkelhülse und der Oberschenkeladapter so bemessen und geformt sind, dass die Oberschenkelhülse auf dem Adapter anbringbar ist, wobei sich der Adapter durch den Kanal der Hülse erstreckt und durch Reibschluss an der Hülse gehalten ist,
**dadurch gekennzeichnet, dass** die Hülse einen einzigen Seitenflügelabschnitt hat, der sich von dem mittigen Bereich erstreckt, so dass die Hülse asymmetrisch ist.

2. Basisausrüstung nach Anspruch 1, bei der die maximale Querabmessung der Hülse (24) an der ersten Querabmessung ist.

3. Basisausrüstung nach Anspruch 1, wobei die Außenkante jeder Terrasse (60a-60h) an dem Übergang des mittigen Bereichs (44) zu dem Seitenflügelbereich (46) zerklüftet ist.

4. Basisausrüstung nach Anspruch 1, bei der die Hülse asymmetrisch um zwei senkrechte Ebenen durch die mittlere Längsachse (48) des Körpers der Hülse (24) ist.

5. Basisausrüstung nach Anspruch 4, die eine zweite Hülse (26, 40) umfasst, wobei die zweite Hülse aufweist:
einen Körper, der eine mittige Längsachse (148), die ein erstes und ein zweites Ende (150, 152) definiert, einen mittleren Bereich (144), einen Seitenflügelbereich (145), einen zweiten Seitenflügelbereich (146) und eine innere Wand (154), die einen Kanal entlang der mittigen Längsachse definiert, aufweist, wobei sich der Kanal zwischen dem ersten und dem zweiten Ende verjüngt,
wobei der Körper eine Außenfläche (150) hat, die eine Vielzahl aneinandergrenzender Terrassen (160a-160h) umfasst, wobei jede Terrasse eine Außenkante (162a-162h) um ihren Umfang hat, wobei die Außenkante jeder Terrasse in einer Ebene liegt, die im Wesentlichen senkrecht zu der mittigen Längsachse des Körpers verläuft,
wobei die Außenkante jeder Terrasse an dem mittleren Bereich des Körpers ein Paar einander diametral gegenüberstehender, gekrümmter Bereiche mit demselben Krümmungsradius aufweist, wobei der Krümmungsmittelpunkt jedes der einander diametral gegenüberstehenden, gekrümmten Bereiche (164a, 164c, 164d, 165a, 165c, 165d) entlang der mittigen Längsachse des Körpers liegt, wobei die einander diametral gegenüberstehenden, gekrümmten Bereiche Scheitelpunkte (166a, 166c, 166d, 167a, 167c, 167d) der Außenkante an dem mittleren Bereich des Körpers definieren,
wobei die Außenkante jeder Terrasse an dem ersten Seitenflügelbereich des Körpers ein paar beabstandete gegenüberstehende gerade Linien (174, 176) aufweist, die mit dem einander diametral gegenüberstehenden, gekrümmten Bereichen verbunden sind und ein gekrümmtes Endsegment (172a, 172c) haben, das von den einander diametral gegenüberstehenden, gekrümmten Bereiche des mittleren Bereiches beabstandet ist, wobei der Abstand zwischen den beabstandeten gegenüberstehenden Linien der Außenkante an dem ersten Seitenflügelbereich kleiner ist als der Abstand zwischen den einander diametral gegenüberstehenden, gekrümmten Bereiche der Außenkante an dem mittleren Bereich des Körpers, wobei das gekrümmte Endsegment einen Scheitelpunkt (178) der Außenkante an dem zweiten Seitenflügelbereich des Körpers definiert,
wobei die Außenkante jeder Terrasse an dem zweiten Seitenflügelbereich des Körpers ein Paar beabstandete gegenüberstehende gerader Linien (174, 176) aufweist, die mit den einander diametral gegenüberstehenden, gekrümmten Bereiche des mittleren Bereiches des Körpers verbunden sind und durch ein gekrümmtes Endsegment (172a, 172c) von den diametral gegenüberstehenden, gekrümmten Bereichen beabstandet sind, wobei der Abstand zwischen den gegenüberstehenden Linien der Außenkante an den zweiten Seitenflügelbereich kleiner ist als der Abstand zwischen den einander diametral gegenüberstehenden, gekrümmten Bereichen der Außenkante an dem mittleren Bereich des Körpers, wobei das gekrümmte Endsegment einen Scheitelpunkt (179) der Außenkante an dem zweiten Seitenflügelbereich des Körpers definiert,
wobei jede Terrasse eine erste Querabmessung ("l") zwischen den Scheitelpunkten der Außenkante an dem ersten Seitenflügelbereich und den zweiten Seitenflügelbereich und eine zweite Querabmessung ("d") zwischen den Scheitelpunkten der einander diametral gegenüberstehenden, gekrümmten Bereiche der Außenkante an dem mittleren Bereich haben,
wobei die erste Querabmessung der Terrasse, die dem ersten Ende am nächsten ist, größer ist als die erste Querabmessung der angrenzenden Terrasse und die zweite Querabmessung der Terrasse, die dem ersten Ende am nächsten liegt, größer ist als die zweite Querabmessung der angrenzenden Terrasse und
wobei die Hülse symmetrisch um zwei senkrechte Ebenen durch die mittige Längsachse des Körpers der Hülse ist.

6. Basisausrüstung nach Anspruch 1, die eine Fräsenführung (400) zur Verwendung beim Bilden einer Höhlung, um die Hülse aufzunehmen, umfasst, wobei die Fräsenführung aufweist:
ein Schablonenelement (402) mit einer Innenfläche (410), die eine längliche Fräsenführungsöffnung definiert, wobei die längliche Fräsenführungsöffnung (412) eine erste Querabmessung und eine zweite Querabmessung hat, wobei die erste Querabmessung der länglichen Führungsöffnung wenigstens so groß ist wie die erste Querabmessung der Terrasse der Hülse, die dem ersten Ende der Hülse am nächsten ist, wobei die zweite Querabmessung kleiner ist als erste Querabmessung,
eine Basis (404), die von dem Schablonenelement beabstandet ist, wobei die Basis eine kugelige Vertiefung (438) umfasst, die mit der Fräsenführungsöffnung ausgerichtet ist, und
eine Seitenwand (406, 408), die das Schablonenelement und das Basiselement verbindet.

7. Basisausrüstung nach Anspruch 6, die einen Probeschaft (450) umfasst, wobei der Probeschaft und die Fräsenführung (400) komplementäre Merkmale zum Verbinden des Probeschaftes mit der Basis (404) der Fräsenführung haben.

8. Basisausrüstung nach Anspruch 7, die eine Räumahle (350) mit einem ersten Ende, einem zweiten Ende und einer mittigen Längsachse umfasst, wobei das erste Ende der Räumahle eine konvexe kugelige Fläche aufweist, die so bemessen und geformt ist, dass sie in der kugeligen Vertiefung (438) der Basis (404) der Fräsenführung (400) aufzunehmen ist, wobei die Räumahle so bemessen und geformt ist, dass sie sich von der Basis der Fräsenführung und durch die längliche Fräsenführungsöffnung (412) erstreckt.

9. Basisausrüstung nach Anspruch 8, die eine zweite Räumahle umfasst, wobei die zweite Räumahle ein erstes Ende, ein zweites Ende, eine mittige Längsachse und einen maximalen Durchmesser hat, wobei der maximale Durchmesser der zweiten Räumahle größer ist als die zweite Querabmessung der länglichen Fräsenführungsöffnung.

10. Basisausrüstung nach Anspruch 9, bei der der mittlere Bereich (44) der Hülse (24, 38) einen maximalen Durchmesser hat und wobei der maximale Durchmesser der zweiten Räumahle derselbe ist, wie der maximale Durchmesser des mittleren Bereiches der Hülse.

11. Basisausrüstung nach Anspruch 8, bei der die kugelige Vertiefung (438) der Basis (404) so bemessen und geformt ist, dass sie die Verschiebebewegung der konvexen kugeligen Fläche des ersten Endes der Räumahle auf eine Länge beschränkt, die kleiner ist als die erste Querabmessung der länglichen Fräsenführungsöffnung (412).

12. Basisausrüstung nach Anspruch 6, bei der der Mittelpunkt der länglichen Fräsenführungsöffnung (412) und der Mittelpunkt der kugeligen Vertiefung (438) der Basis (404) auf einer Längsachse liegen.

## Revendications

1. Kit de prothèse du genou modulaire comprenant :
(a) un composant fémoral (10) comportant des surfaces condylaires courbes convexes (12, 14),
(b) un composant tibial (30) comportant des surfaces condylaires courbes concaves pour recevoir les surfaces condylaires courbes convexes du composant fémoral, les surfaces condylaires courbes concaves du composant tibial et les surfaces condylaires courbes convexes du composant fémoral définissant une surface de mouvement articulaire, et
(c) un manchon (24, 38) comprenant un corps ayant un axe longitudinal central (48) qui définit des première et deuxième extrémités (50, 52), une partie centrale (44), une partie à ailette latérale (46) s'étendant de la partie centrale et une paroi interne (54) définissant un canal (56) le long de l'axe longitudinal central, le canal s'effilant entre les première et deuxième extrémités, dans lequel le corps a une surface externe (58) qui comprend une pluralité de terrasses adjacentes (60a-60b), chaque terrasse ayant un bord extérieur (62a-62h) autour de son périmètre, le bord externe de chaque terrasse reposant dans un plan substantiellement perpendiculaire à l'axe longitudinal central du corps,
dans lequel le bords externe de chaque terrasse au niveau de la partie centrale du corps comprend une paire de parties courbes diamétralement opposées (64a, 64c, 64d, 65c, 65d) définissant des sommets du bord externe sur la partie centrale du corps, et le bord externe de chaque terrasse sur la partie à ailette latérale du corps étant raccordé aux parties courbes diamétralement opposées et ayant un segment terminal courbe (72a, 72c) espacé des parties courbes diamétralement opposées de la partie centrale, le segment terminal courbe définissant un sommet (78) du bord externe sur la partie à ailette latérale du corps, chaque terrasse ayant une première dimension transversale ("l") qui est la dimension la plus grande de la terrasse et une deuxième dimension transversale ("d") entre les sommets des parties courbes diamétralement opposées du bord externe sur la partie centrale, la première dimension transversale de la terrasse la plus proche de la première extrémité étant supérieure à la première dimension transversale de la terrasse adjacente et la deuxième dimension transversale de la terrasse la plus proche de la première extrémité étant supérieure à la deuxième dimension transversale de la terrasse adjacente,
dans lequel (i) le composant tibial comprend un plateau tibial (30) ayant une tige (36) et un insert tibial (32) porté par le plateau tibial, et dans lequel le manchon définit un manchon tibial, le manchon tibial et la tige étant dimensionnés et formés de sorte que le manchon tibial puisse être monté sur la tige, la tige s'étendant dans le canal du manchon et étant bloquée par frottement au manchon, ou (ii) le kit comprend un adaptateur fémoral (16) pouvant être monté sur le composant fémoral (10) et dans lequel le manchon définit un manchon fémoral, le manchon fémoral et l'adaptateur fémoral étant dimensionnés et formés de telle sorte que le manchon fémoral puisse être monté sur l'adaptateur, l'adaptateur s'étendant dans le canal du manchon et étant bloqué par frottement au manchon,
**caractérisé en ce que** le manchon a une partie à ailette latérale unique s'étendant de la partie centrale de sorte que le manchon soit asymétrique.

2. Kit selon la revendication 1, dans lequel la dimension transversale maximale du manchon (24) est à la première dimension transversale.

3. Kit selon la revendication 1, dans lequel le bord externe de chaque terrasse (60a-60h) est indenté à la jonction de la partie centrale (44) et de la partie d'ailette latérale (46).

4. Kit selon la revendication 1, dans lequel le manchon est asymétrique autour de deux plans perpendiculaires dans l'axe longitudinal central (48) du corps du manchon (24).

5. Kit selon la revendication 4, qui comprend un deuxième manchon (26, 40), le deuxième manchon comprenant :
✔ un corps ayant un axe longitudinal central (148) qui définit des première et deuxième extrémités (150, 152), une partie centrale (144), une première partie à ailette latérale (145), une deuxième partie à ailette latérale (146) et une paroi interne (154) définissant un canal (156) le long de l'axe longitudinal central, le canal s'effilant entre les première et deuxièmes extrémités,
✔ ledit corps ayant une surface externe (158) qui comprend une pluralité de terrasses adjacentes (160a-160h), chaque terrasse ayant un bord externe (162a-162h) autour de son périmètre, le bord externe de chaque terrasse reposant dans un plan substantiellement perpendiculaire à l'axe longitudinal central du corps,
✔ le bord externe de chaque terrasse sur la partie centrale du corps comprenant une paire de parties courbes diamétralement opposées ayant le même rayon de courbure, le centre de courbure de chacune des parties courbes diamétralement opposées (164a, 164c, 164d, 165a, 165c, 165d) étant le long de l'axe central longitudinal du corps, les parties courbes diamétralement opposées définissant des sommets (166a, 166c, 166d, 167a, 167c, 167d) du bord externe sur la partie centrale du corps,
✔ le bord externe de chaque terrasse sur la première partie à ailette latérale du corps comprenant une paire de lignes droites opposées espacées (174, 176) raccordées aux parties courbes diamétralement opposées et ayant un segment terminal courbe (172a, 172c) espacé des parties courbes diamétralement opposées de la partie centrale, la distance entre les lignes opposées espacées du bord externe sur la première partie à ailette latérale étant inférieure à la distance entre les parties courbes diamétralement opposées du bord externe sur la partie centrale du corps, le segment terminal courbe définissant un sommet (178) du bord externe sur la deuxième partie à ailette latérale du corps,
✔ le bord externe de chaque terrasse sur la deuxième partie d'ailette latérale du corps comprenant une paire de lignes droites opposées espacées (174, 176) raccordées aux parties courbes diamétralement opposées de la partie centrale du corps et par un segment terminal courbe (172a, 172c) espacé des parties courbes diamétralement opposées, la distance entre les lignes opposées du bord externe à la deuxième partie d'ailette latérale étant inférieure à la distance entre les parties courbes diamétralement opposées du bord externe sur la partie centrale du corps, le segment terminal courbe définissant un sommet (179) du bord externe sur la deuxième partie d'ailette latérale du corps,
✔ chaque terrasse ayant une première dimension transversale ("l") entre les sommets du bord externe sur la première partie d'ailette latérale et une deuxième partie d'ailette latérale et une deuxième dimension transversale ("d") entre les sommets des parties courbes diamétralement opposées du bord externe sur la partie centrale,
✔ dans lequel la première dimension transversale de la terrasse la plus proche de la première extrémité est supérieure à la première dimension transversale de la terrasse adjacente et la deuxième dimension transversale de la terrasse la plus proche de la première extrémité est supérieure à la deuxième dimension transversale de la terrasse adjacente, et
✔ dans lequel le manchon est symétrique autour de deux plans perpendiculaires par l'axe longitudinal central du corps du manchon.

6. Kit selon la revendication 1, qui comprend un guide de fraisage (400) pour l'utilisation dans la formation d'une cavité destinée à recevoir le manchon, le guide de fraisage comprenant :
✔ un élément matrice (402) ayant une surface interne (410) définissant une ouverture de guide de fraisage allongé, l'ouverture de guide de fraisage allongé (412) ayant une première dimension transversale et une deuxième dimension transversale, la première dimension transversale de l'ouverture de guide de fraisage allongé étant au moins aussi grande que la première dimension transversale de la terrasse du manchons le plus proche de la première extrémité du manchon, la deuxième dimension transversale étant inférieure à la première dimension transversale,
✔ une base (404) espacée de l'élément matrice, la base comprenant une dépression sphérique (438) alignée avec l'ouverture du guide de fraisage, et
✔ une paroi latérale (406, 408) raccordant l'élément matrice et l'élément de base.

7. Kit selon la revendication 6, qui comprend un essai de tige (450), l'essai de tige et le guide de fraisage (400) ayant des caractéristiques complémentaires pour raccorder l'essai de tige à la base (404) du guide de fraisage.

8. Kit selon la revendication 7, qui comprend un alésoir (350) ayant une première extrémité, une deuxième extrémité et un axe longitudinal central, la première extrémité de l'alésoir comprenant une surface sphérique convexe dimensionnée et formée pour être reçue dans la dépression sphérique (438) de la base (404) du guide de fraisage (400), l'alésoir étant dimensionné et formé pour s'étendre de la base du guide de fraisage et dans l'ouverture du guide de fraisage allongé (412).

9. Kit selon la revendication 8, qui comprend un deuxième alésoir, le deuxième alésoir ayant une première extrémité, une deuxième extrémité, un axe longitudinal central et un diamètre maximal, dans lequel le diamètre maximal du deuxième alésoir est supérieur à la deuxième dimension transversale de l'ouverture du guide de fraisage allongé.

10. Kit selon la revendication 9, dans lequel la partie centrale (44) du manchon (24, 38) a un diamètre maximal et dans lequel le diamètre maximal du deuxième alésoir est le même que le diamètre maximal de la partie centrale du manchon.

11. Kit selon la revendication 8, dans lequel la dépression sphérique (438) de la base (404) est dimensionnée et formée de façon à limiter un mouvement en translation de la surface sphérique convexe de la première extrémité de l'alésoir à une distance inférieure à la première dimension transversale de l'ouverture du guide de fraisage allongé (412).

12. Kit selon la revendication 6, dans lequel le centre de l'ouverture du guide de fraisage allongé (412) et le centre de la dépression sphérique (438) de la base (404) se situent sur un axe longitudinal.
